# EUROPEAN PATENT SPECIFICATION

(11) **EP 3 296 407 B1**
(45) Date of publication and mention of the grant of the patent: **22.05.2019**
(21) Application number: 17197306.8
(22) Date of filing: 18.02.2005
(51) Int. Cl.: C12Q 1/6886

(54) **MUTATIONS OF THE PIK3CA GENE IN HUMAN CANCERS**
MUTATIONEN DES PIK3CA-GENS IN MENSCHLICHEN KREBSEN
MUTATIONS DU GÈNE PIK3CA DANS LES CANCERS HUMAINS

(30) Priority: 02.03.2004 US 548886 P
(43) Date of publication of application: 21.03.2018
(62) Divisional of application: 15150891.8
(73) Proprietor: The Johns Hopkins University, Baltimore, MD 21218 (US)
(72) Inventor: SAMUELS, Yardena, Potomac, MD 20854 (US); VELCULESCU, Victor, Dayton, MD 21036 (US); KINZLER, Kenneth, Baltimore, MD 21230-3959 (US); VOGELSTEIN, Bert, Baltimore, MD 21208 (US)
(74) Representative: Tombling, Adrian George

(56) References cited:
- PHILP AMANDA J ET AL: "The phosphatidylinositol 3'-kinase p85alpha gene is an oncogene in human ovarian and colon tumors", CANCER RESEARCH, AACR - AMERICAN ASSOCIATION FOR CANCER RESEARCH, US, vol. 61, no. 20, 15 October 2001 (2001-10-15), pages 7426-7429, XP002505603, ISSN: 0008-5472
- ZHANG LIN ET AL: "The oncogene phosphatidylinositol 3'-kinase catalytic subunit alpha promotes angiogenesis via vascular endothelial growth factor in ovarian carcinoma.", CANCER RESEARCH, vol. 63, no. 14, 15 July 2003 (2003-07-15) , pages 4225-4231, XP002539224, ISSN: 0008-5472
- MA Y-Y ET AL: "PIK3CA as an oncogene in cervical cancer", ONCOGENE, NATURE PUBLISHING GROUP, GB BASINGSTOKE, HANTS, vol. 19, no. 23, 25 May 2000 (2000-05-25), pages 2739-2744, XP002459619, ISSN: 0950-9232
- SHAYESTEH LALEH ET AL: "PIK3CA is implicated as an oncogene in ovarian cancer", NATURE GENETICS, vol. 21, no. 1, January 1999 (1999-01), pages 99-102, XP002539225, ISSN: 1061-4036

## Description

### FIELD OF THE INVENTION

'The invention relates to the fields of diagnostic tests and therapeutic methods for cancer.

### BACKGROUND OF THE INVENTION

PI3Ks are lipid kinases that function as signal transducers downstream of cell surface receptors and mediate pathways important for cell growth, proliferation, adhesion, survival and motility *(1*, *2)*. Although increased PI3K activity has been observed in marry colorectal and other tumors (3, 4), no intragenic mutations of PI3K have been identified.

Members of the PIK3 pathway have been previously reported to be altered in cancers, for example, the PTEN tumor suppressor gene (75, 76), whose function is to reverse the phosphorylation mediated by PI3Ks (77, *IS).* Reduplication or amplification of the chromosomal regions containing PIK3CA and AKT2 has been reported in some human cancers (2, *19*, *20),* but the genes that are the targets of such large-scale genetic events have not been and cannot easily be defined.

Philip et al., Cancer Res 2001 Oct 15; 61(20): 7426-7429 discloses the detection of nucleotide mutations in the p85α subunit of PI3K but there is no reference to PIK3CA.

### BRIEF SUMMARY OF THE INVENTION

In a first embodiment a method is provided for detecting a mutated PIK3CA polynucleotide in accordance with claim 1.

In a second embodiment of the invention a method is provided for detecting a mutated PIK3CA polynucleotide in accordance with claim 2.

### BRIEF DESCRIPTION OF THE DRAWINGS

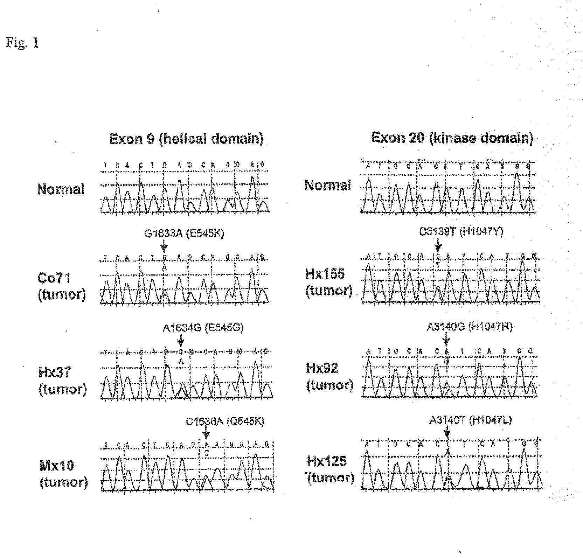

Fig. 1. Detection of mutations in of PIK3CA, Representative examples of mutations in exons 9 and 20. In each case, the top sequence chromatogram was obtained from normal tissue and the three lower sequence chromatograms from the indicated tumors, Arrows indicate the location of missense mutations. The nucleotide and amino acid alterations are indicated above the arrow.

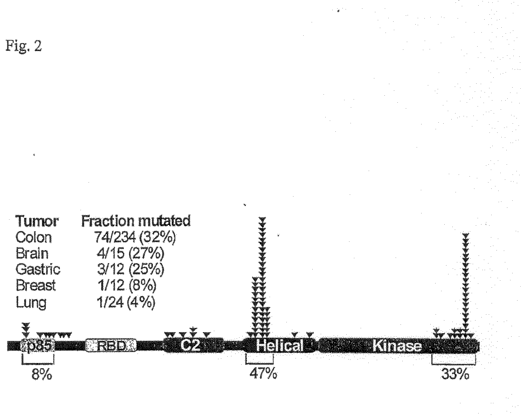

Fig. 2, Distribution of mutations in PIK3CA. Arrows indicate the location of missense mutations, and boxes represent functional domains (p85BD, p85 binding domain; RBD, Ras binding domain; C2 domain; Helical domain; Kinase domain). The percentage of mutations detected within each region in cancers is indicated below.

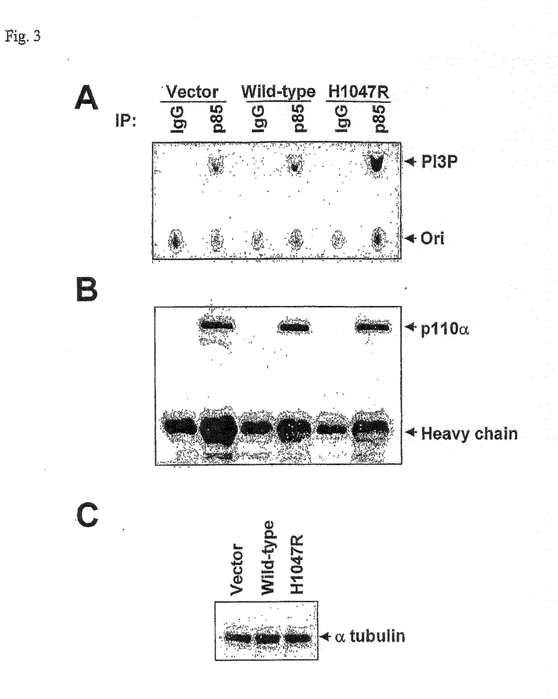

Figs. 3A-3C. Increased lipid kinase activity of mutant p110α. NIH3T3 cells were transfected with empty vector or with vector constructs containing either wild-type p110α or mutant p110α (H1047R) as indicated above the lanes. Immunoprecipitations were performed either with control IgG or anti-p85 polyclonal antibodies. (Fig. 3A) Half of the immunoprecipitates were subjected to a PI3-kinase assay using phosphatidylinositol as a substrate. "PI3P" indicates the position of PI-3-phosphate determined with standard phosphatidyl markers and "Ori" indicates the origin. (Fig. 3B) The other half of the immunoprecipitates was analyzed by western blotting with anti-p110α antibody. (Fig. 3C) Cell lysates from transfected cells contained similar amounts of total protein as determined by western blotting using an anti-α-tubulin antibody. Identical results to those shown in this figure were observed in three independent transfection experiments.

### DETAILED DESCRIPTION OF THE INVENTION

The clustering of mutations within PIK3CA make it an excellent marker for early detection or for following disease progression. Testing focused in the clustered regions will yield most of the mutant alleles.

The human PIK3CA coding sequence is reported in the literature and is shown in SEQ ID NO: 1. This is the sequence of one particular individual in the population of humans. Humans vary from one to another in their gene sequences. These variations are very minimal, sometimes occurring at a frequency of about 1 to 10 nucleotides per gene. Different forms of any particular gene exist within the human population. These different forms are called allelic variants. Allelic variants often do not change the amino acid sequence of the encoded protein; such variants are termed synonymous. Even if they do change the encoded amino acid (non-synonymous), the function of the protein is not typically affected. Such changes are evolutionarily or functionally neutral. When human PIK3CA is referred to in the present application all allelic variants are intended to be encompassed by the term. The sequence of SEQ ID NO: 1 is provided merely as a representative example of a wild-type human sequence. The invention is not limited to this single allelic form of PIK3CA. For purposes of determining a mutation, PIK3CA sequences determined in a test sample can be compared to a sequence determined in a different tissue of the human. A difference in the sequence in the two tissues indicates a somatic mutation. Alternatively, the sequence determined in a PIK3CA gene in a test sample can be compared to the sequence of SEQ ID NO: 1. A difference between the test sample sequence and SEQ ID NO: 1 can be identified as a mutation. Tissues suspected of being cancerous can be tested, as can body samples that may be expected to contain sloughed-off cells from tumors or cells of cancers. Suitable body samples for testing include blood, serum, plasma, sputum, urine, stool, nipple aspirate, saliva, and cerebrospinal fluid.

Mutations in PIK3CA cluster in exons 9 (SEQ ID NO: 4) and 20 (SEQ ID NO: 5). Other mutations occur, but these two exons appear to be the hotspots for mutations. Many mutations occur in PIK3CA's helical domain (nt 1567-2124 of SEQ ID NO: 2) and in its kinase domain (nt 2095-3096 of SEQ ID NO: 2). Fewer occur in PIK3CA's P85BD domain (nt 103-335 of SEQ ID NO: 2). Mutations have been found in exons 1, 2, 4, 5, 7, 9, 13, 18, and 20. Any combination of these exons can be tested, optionally in conjunction with testing other exons. Testing for mutations can be done along the whole coding sequence or can be focused in the areas where mutations have been found to cluster. Particular hotspots of mutations occur at nucleotide positions 1624, 1633, 1636, and 3140 of PIK3CA coding sequence.

PIK3CA mutations have been found in a variety of different types of tumors. Thus any of a variety of tumors can be tested for PIK3CA mutations. These tissues include, without limitation: colorectal tissue, brain tissue, gastric tissue, breast tissue, and lung tissue.

Any type of intragenic mutation can be detected. These include substitution mutations, deletion mutations, and insertion mutations. The size of the mutations is likely to be small, on the order of from 1 to 3 nucleotides. Mutations which can be detected include, but are not limited to G1624A, G1633A, C1636A, A3140G, G113A, T1258C, G3129T, C3139T, and G2702T. Any combination of these mutations can be tested.

The mutations that are found in PIK3CA appear to be activating mutations. Thus therapeutic regimens involving inhibition of p110α activity or expression can be used to inhibit progression of a tumor in a human. Inhibitory molecules which can be used include antisense oligonucleotides or antisense constructs, a molecule comprising an antibody binding region, and siRNA molecules. Molecules comprising an antibody binding region can be full antibodies, single chain variable regions, antibody fragments, antibody conjugates, etc. The antibody binding regions may but need not bind to epitopes contained within the kinase domain (nt 2095-3096 of SEQ ID NO: 2) of PIK3CA, the helical domain (nt 1567-2124 of SEQ ID NO: 2) of PIK3CA, or the P85BD domain (nt 103-335 of SEQ ID NO: 2) of PIK3CA.

Antisense constructs, antisense oligonucleotides, RNA interference constructs or siRNA duplex RNA molecules can be used to interfere with expression of PIK3CA. Typically at least 15, 17, 19, or 21 nucleotides of the complement of PIK3CA mRNA sequence are sufficient for an antisense molecule. Typically at least 19, 21, 22, or 23 nucleotides of PIK3CA are sufficient for an RNA interference molecule. Preferably an RNA interference molecule will have a 2 nucleotide 3' overhang, If the RNA interference molecule is expressed in a cell from a construct, for example from a hairpin molecule or from an inverted repeat of the desired PIK3CA sequence, then the endogenous cellular machinery will create the overhangs. siRNA molecules can be prepared by chemical synthesis, in vitro transcription, or digestion of long dsRNA by Rnase III or Dicer. These can be introduced into cells by transfection, electroporation, or other methods known in the art. See Hannon, GJ, 2002, RNA Interference, Nature 418: 244-251; Bernstein E et al., 2002, The rest is silence. RNA 7: 1509-1521; Hutvagner G et al., RNAi: Nature abhors a double-strand. Curr. Opin. Genetics & Development 12: 225-232; Brummelkamp, 2002, A system for stable expression of short interfering RNAs in mammalian cells. Science 296: 550-553; Lee NS, Dohjima T, Bauer G, Li H, Li M-J, Ehsani A, Salvaterra P, and Rossi J. (2002). Expression of small interfering RNAs targeted against HIV-1 rev transcripts in human cells. Nature Biotechnol. 20:500-505; Miyagishi M, and Taira K. (2002). U6-promoter-driven siRNAs with four uridine 3' overhangs efficiently suppress targeted gene expression in mammalian cells. Nature Biotechnol. 20:497-500; Paddison PJ, Caudy AA, Bernstein E, Hannon GJ, and Conklin DS. (2002). Short hairpin RNAs (shRNAs) induce sequence-specific silencing in mammalian cells. Genes & Dev. 16:948-958; Paul CP, Good PD, Winer I, and Engelke DR. (2002). Effective expression of small interfering RNA in human cells. Nature Biotechnol. 20:505-508; Sui G, Soohoo C, Affar E-B, Gay F, Shi Y, Forrester WC, and Shi Y. (2002). A DNA vector-based RNAi technology to suppress gene expression in mammalian cells. Proc. Natl. Acad. Sci. USA 99(6):5515-5520; Yu J-Y, DeRuiter SL, and Turner DL. (2002). RNA interference by expression of short-interfering RNAs and hairpin RNAs in mammalian cells. Proc. Natl. Acad. Sci. USA 99(9):6047-6052.

Antisense or RNA interference molecules can be delivered in *vitro* to cells or *in vivo, e.g.,* to tumors of a mammal. Typical delivery means known in the art can be used. For example, delivery to a tumor can be accomplished by intratumoral injections. Other modes of delivery can be used without limitation, including: intravenous, intramuscular, intraperitoneal, intraarterial, local delivery during surgery, endoscopic, subcutaneous, and *per os.* In a mouse model, the antisense or RNA interference can be adminstered to a tumor cell *in vitro,* and the tumor cell can be subsequently administered to a mouse. Vectors can be selected for desirable properties for any particular application. Vectors can be viral or plasmid. Adenoviral vectors are useful in this regard. Tissue-specific, cell-type specific, or otherwise regulatable promoters can be used to control the transcription of the inhibitory polynucleotide molecules. Non-viral carriers such as liposomes or nanospheres can also be used.

Using the p110α protein herein disclosed, one of ordinary skill in the art can readily generate antibodies which specifically bind to the proteins. Such antibodies can be monoclonal or polyclonal. They can be chimeric, humanized, or totally human. Any functional fragment or derivative of an antibody can be used including Fab, Fab', Fab2, Fab'2, and single chain variable regions. So long as the fragment or derivative retains specificity of binding for the endothelial marker protein it can be used. Antibodies can be tested for specificity of binding by comparing binding to appropriate antigen to binding to irrelevant antigen or antigen mixture under a given set of conditions. If the antibody binds to the appropriate antigen at least 2, 5, 7, and preferably 10 times more than to irrelevant antigen or antigen mixture then it is considered to be specific.

Techniques for making such partially to fully human antibodies are known in the art and any such techniques can be used. According to one particularly preferred embodiment, fully human antibody sequences are made in a transgenic mouse which has been engineered to express human heavy and light chain antibody genes. Multiple strains of such transgenic mice have been made which can produce different classes of antibodies. B cells from transgenic mice which are producing a desirable antibody can be fused to make hybridoma cell lines for continuous production of the desired antibody. See for example, Nina D. Russel, Jose R. F. Corvalan, Michael L. Gallo, C. Geoffrey Davis, Liise-Anne Pirofski. Production of Protective Human Antipneumococcal Antibodies by Transgenic Mice with Human Immunoglobulin Loci Infection and Immunity April 2000, p. 1820-1826; Michael L. Gallo, Vladimir E. Ivanov, Aya Jakobovits, and C. Geoffrey Davis. The human immunoglobulin loci introduced into mice: V (D) and J gene segment usage similar to that of adult humans European Journal of Immunology 30: 534-540, 2000; Larry L. Green. Antibody engineering via genetic engineering of the mouse: XenoMouse strains are a vehicle for the facile generation of therapeutic human monoclonal antibodies Journal of Immunological Methods 231 11-23, 1999; Yang X-D, Corvalan JRF, Wang P, Roy CM-N and Davis CG. Fully Human Anti-interleukin-8 Monoclonal Antibodies: Potential Therapeutics for the Treatment of Inflammatory Disease States. Journal of Leukocyte Biology Vol. 66, pp401-410 (1999); Yang X-D, Jia X-C, Corvalan JRF, Wang P, CG Davis and Jakobovits A. Eradication of Established Tumors by a Fully Human Monoclonal Antibody to the Epidermal Growth Factor Receptor without Concomitant Chemotherapy. Cancer Research Vol. 59, Number 6, pp1236-1243 (1999) ; Jakobovits A. Production and selection of antigen-specific fully human monoclonal antibodies from mice engineered with human Ig loci. Advanced Drug Delivery Reviews Vol. 31, pp: 33-42 (1998); Green L and Jakobovits A. Regulation of B cell development by variable gene complexity in mice reconstituted with human immunoglobulin yeast artificial chromosomes. J. Exp. Med. Vol. 188, Number 3, pp: 483-495 (1998); Jakobovits A. The long-awaited magic bullets: therapeutic human monoclonal antibodies from transgenic mice. Exp. Opin. Invest. Drugs Vol. 7(4), pp : 607-614 (1998) ; Tsuda H, Maynard-Currie K, Reid L, Yoshida T, Edamura K, Maeda N, Smithies O, Jakobovits A. Inactivation of Mouse HPRT locus by a 203-bp retrotransposon insertion and a 55-kb gene-targeted deletion: establishment of new HPRT-Deficient mouse embryonic stem cell lines. Genomics Vol. 42, pp: 413-421 (1997); Sherman-Gold, R. Monoclonal Antibodies: The Evolution from '80s Magic Bullets To Mature, Mainstream Applications as Clinical Therapeutics. Genetic Engineering News Vol. 17, Number 14 (August 1997); Mendez M, Green L, Corvalan J, Jia X-C, Maynard-Currie C, Yang X-d, Gallo M, Louie D, Lee D, Erickson K, Luna J, Roy C, Abderrahim H, Kirschenbaum F, Noguchi M, Smith D, Fukushima A, Hales J, Finer M, Davis C, Zsebo K, Jakobovits A. Functional transplant of megabase human immunoglobulin loci recapitulates human antibody response in mice. Nature Genetics Vol. 15, pp: 146-156 (1997); Jakobovits A. Mice engineered with human immunoglobulin YACs: A new technology for production of fully human antibodies for autoimmunity therapy. Weir's Handbook of Experimental Immunology, The Integrated Immune System Vol. IV, pp: 194.1-194.7 (1996) ; Jakobovits A. Production of fully human antibodies by transgenic mice. Current Opinion in Biotechnology Vol. 6, No. 5, pp: 561-566 (1995) ; Mendez M, Abderrahim H, Noguchi M, David N, Hardy M, Green L, Tsuda H, Yoast S, Maynard-Currie C, Garza D, Gemmil1 R, Jakobovits A, Klapholz S. Analysis of the structural integrity of YACs comprising human immunoglobulin genes in yeast and in embryonic stem cells. Genomics Vol. 26, pp: 294-307 (1995); Jakobovits A. YAC Vectors: Humanizing the mouse genome. Current Biology Vol. 4, No. 8, pp: 761-763 (1994); Arbones M, Ord D, Ley K, Ratech H, Maynard-Curry K, Otten G, Capon D, Tedder T. Lymphocyte homing and leukocyte rolling and migration are impaired in L-selectin-deficient mice. Immunity Vol. 1, No. 4, pp: 247-260 (1994); Green L, Hardy M, Maynard-Curry K, Tsuda H, Louie D, Mendez M, Abderrahim H, Noguchi M, Smith D, Zeng Y, et. al. Antigen-specific human monoclonal antibodies from mice engineered with human Ig heavy and light chain YACs. Nature Genetics Vol. 7, No. 1, pp: 13-21 (1994); Jakobovits A, Moore A, Green L, Vergara G, Maynard-Curry K, Austin H, Klapholz S. Germ-line transmission and expression of a human-derived yeast artificial chromosome. Nature Vol. 362, No. 6417, pp: 255-258 (1993) ; Jakobovits A, Vergara G, Kennedy J, Hales J, McGuinness R, Casentini-Borocz D, Brenner D, Otten G. Analysis of homozygous mutant chimeric mice: deletion of the immunoglobulin heavy-chain joining region blocks B-cell development and antibody production. Proceedings of the National Academy of Sciences USA Vol. 90, No. 6, pp: 2551-2555 (1993); Kucherlapati et al., U.S. 6,1075,181.

Antibodies can also be made using phage display techniques. Such techniques can be used to isolate an initial antibody or to generate variants with altered specificity or avidity characteristics. Single chain Fv can also be used as is convenient. They can be made from vaccinated transgenic mice, if desired, Antibodies can be produced in cell culture, in phage, or in various animals, including but not limited to cows, rabbits, goats, mice, rats, hamsters, guinea pigs, sheep, dogs, cats, monkeys, chimpanzees, apes.

Antibodies can be labeled with a detectable moiety such as a radioactive atom, a chromophore, a fluorophore, or the like. Such labeled antibodies can be used for diagnostic techniques, either *in vivo,* or in an isolated test sample. Antibodies can also be conjugated, for example, to a pharmaceutical agent, such as chemotherapeutic drug or a toxin. They can be linked to a cytokine, to a ligand, to another antibody. Suitable agents for coupling to antibodies to achieve an anti-tumor effect include cytokines, such as interleukin 2 (IL-2) and Tumor Necrosis Factor (TNF); photosensitizers, for use in photodynamic therapy, including aluminum (III) phthalocyanine tetrasulfonate, hematoporphyrin, and phthalocyanine; radionuclides, such as iodine-131 (¹³¹I), yttrium-90 (⁹⁰Y), bismuth-212 (²¹²Bi), bismuth-213 (²¹³Bi), technetium-99m (^{99m}Tc), rhenium-186 (¹⁸⁶Re), and rhenium-188 (¹⁸⁸Re); antibiotics, such as doxorubicin, adriamycin, daunorubicin, methotrexate, daunomycin, neocarzinostatin, and carboplatin; bacterial, plant, and other toxins, such as diphtheria toxin, pseudomonas exotoxin A, staphylococcal enterotoxin A, abrin-A toxin, ricin A (deglycosylated ricin A and native ricin A), TGF-alpha toxin, cytotoxin from Chinese cobra (naja naja atra), and gelonin (a plant toxin); ribosome inactivating proteins from plants, bacteria and fungi, such as restrictocin (a ribosome inactivating protein produced by *Aspergillus rextrictus*), saporin (a ribosome inactivating protein from *Saponaria officinalis*), and RNase; tyrosine kinase inhibitors; ly207702 (a difluorinated purine nucleoside); liposomes containing antitumor agents (*e.g.,* antisense oligonucleotides, plasmids which encode for toxins, methotrexate, etc.); and other antibodies or antibody fragments, such as F(ab).

Those of skill in the art will readily understand and be able to make such antibody derivatives, as they are well known in the art. The antibodies may be cytotoxic on their own, or they may be used to deliver cytotoxic agents to particular locations in the body. The antibodies can be administered to individuals in need thereof as a form of passive immunization.

Given the success of small molecule protein kinase inhibitors, one can develop specific or non-specific inhibitors of p110α for treatment of the large number of patients with these mutations or cancers generally. It is clearly possible to develop broad-spectrum PI3K inhibitors, as documented by studies of LY294002 and wortmannin (*2*, *21,22*)*.* Our data suggest that the development of more specific inhibitors that target p110α but not other PI3Ks would be worthwhile.

Candidate chemotherapeutic agents can be identified as agents which inhibit p110α activity or expression. Test compounds can be synthetic or naturally occurring. They can be previously identified to have physiological activity or not. Tests on candidate chemotherapeutic agents can be run in cell-free systems or in whole cells. p110α activity can be tested by any means known in the art. These include methods taught in references 2, 22 and in Truitt et al., J. Exp. Med., 179, 1071-1076 (1994). Expression can be monitored by determining PI3KCA protein or mRNA. Antibody methods such as western blotting can be used to determine protein. Northern blotting can be used to measure mRNA. Other methods can be used without limitation. When testing for chemotherapeutic agents, the p110α used in the assay can be a wild-type or an activated form. The activated form may contain a substitution mutation selected from the group consisting of E542K, E545K, Q546K, and H1047R. Moreover, inhibitors can be tested to determine their specificity for either p110α or an activated form of p110α. Comparative tests can be run against similar enzymes including PIK3CB, PIK3CG, PIK3C2A, PIK3C2B, PIK3C2G, PIK3C3, A-TM, ATR, FRAP1, LAT1-3TM,SMG1, PRKDC, and TRRAP to determine the relative specificity for the p110α enzyme.

Once a non-synonymous, intragenic mutation in a PIK3CA coding sequence is identified in a test tissue of a patient, that information can be used to make therapeutic decisions. Patients with such mutations are good candidates for therapy with a p110α inhibitor. Such inhibitors can be specific or general for the family of inhibitors. Such inhibitors include LY294002 and wortmannin. Such inhibitors further include molecules comprising an antibody binding region specific for p110α. Such molecules are discussed above.

Sets of primers for amplifying and/or sequencing PIK3CA can be provided in kits or assembled from components. Useful sets include pairs of forward and reverse primers optionally teamed with sequencing primers. The forward primers are shown in SEQ ID NO: 6 to 158. The reverse primers are shown in SEQ ID NO: 159 to 310. The sequencing primers are shown in : SEQ ID NO: 311 to 461. Pairs or triplets or combinations of these pairs or triplets can be packaged and used together to amplify and/or sequence parts of the PIK3CA gene. Pairs can be packaged in single or divided containers. Instructions for using the primers according to the methods of the present invention can be provided in any medium which is convenient, including paper, electronic, or a world-wide web address.

### EXAMPLES

### Example 1-This example demonstrates that the PIK3CA gene is the predominant target of mutations in this gene family

To evaluate whether PI3Ks is genetically implicated in tumorigenesis, we directly examined the DNA sequences of members of this gene family in colorectal cancers.

PI3K catalytic subunits are divided into three major classes depending on their substrate specificity (5). Additionally, a set of more distantly related proteins, including members of the mTOR family, constitute a fourth class (*6*). We used Hidden Markov models to identify 15 human genes containing kinase domains related to those of known PI3Ks in the human genome (*7*). These comprised seven PI3Ks, six members of the mTOR subfamily and two uncharacterized PI3K-like genes (Table 1).

**Table 1. PI3K genes analyzed**

| **Gene name** | **Celera Accession** | **Genbank Accession** | **Alternate names** | **Group*** |
|---|---|---|---|---|
| *PIK3CA* | hCT1640694 | NM_006218 | p110-alpha | Class IA |
| *PIK3CB* | hCT7084 | NM_006219 | PIK3C1, p110-beta | Class IA |
| *PIK3CD* | hCT2292011 | NM_005026 | p110-delta | Class IA |
| *PIK3CG* | hCT7976 | NM_002649 | PI3CG, PI3K-gamma | Class IB |
| *PIK3C2A* | hCT2270768 | NM_002645 | CPK, PI3-K-C2A, PI3K-C2alpha | Class II |
| *PIK3C2B* | hCT7448 | NM_002646 | C2-PI3K, PI3K-C2beta | Class II |
| *PIK3C2G* | hCT1951422 | NM_004570 | PI3K-C2-gamma | Class II |
| *PIK3C3* | hCT13660 | NM_002647 | Vps34 | Class III |
| *ATM* | hCT29277 | NM_000051 | AT1, ATA, ATC, ATD, ATE, ATDC | Class IV |
| *ATR* | hCT1951523 | NM_001184 | FRP1, SCKL, SCKL1 | Class IV |
| *FRAP1* | hCT2292935 | NM_004958 | FRAP, MTOR, FRAP2, RAFT1, RAPT1 | Class IV |
| *SMG1* | hCT2273636 | NM_014006 | ATX, LIP, KIAA0421 | Class IV |
| *PRKDC* | hCT2257127 | NM_006904 | p350, DNAPK, DNPK1, HYRC1, XRCC7 | Class IV |
| *TRRAP* | hCT32594 | NM_003496 | TR-AP, PAF400 | Class IV |
| none | hCT2257641 | none | | Class IV |
| none | hCT13051 | none | | Class IV |

| | | | | |
|---|---|---|---|---|
| *PI3K genes are grouped into previously described classes (S3,S4). Class I, II and III comprise PI3K catalytic subunits, white class IV comprises PI3K-like genes including members of the mTOR (target of rapamycin), ATM (ataxia telangiectasia mutated), and DNAPK (DNA-dependent protein kinase) subfamilies, as well as two previously uncharacterized genes. | | | | |

We initially examined 111 exons encoding the predicted kinase domains of these genes (Table 2). The exons were polymerase chain reaction (PCR) amplified and directly sequenced from genomic DNA of 35 colorectal cancers (*8*). Only one of the genes (PIK3CA) contained any somatic (*i.e*., tumor-specific) mutations.

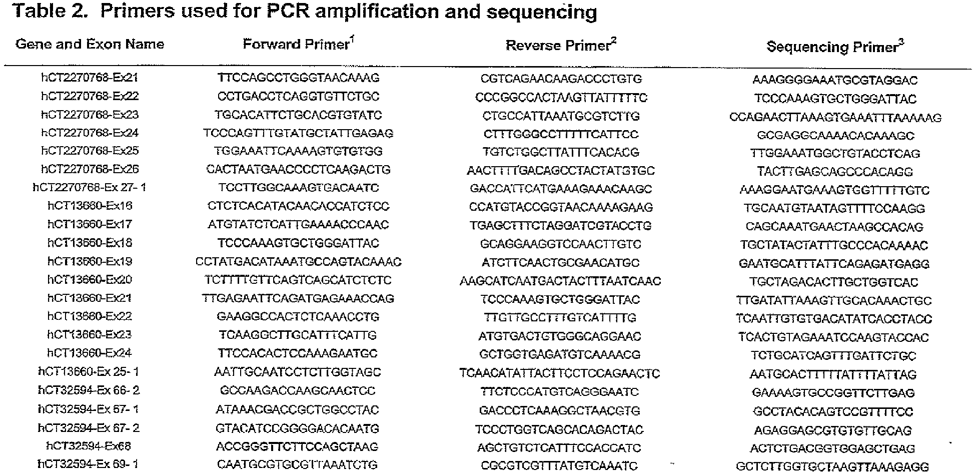

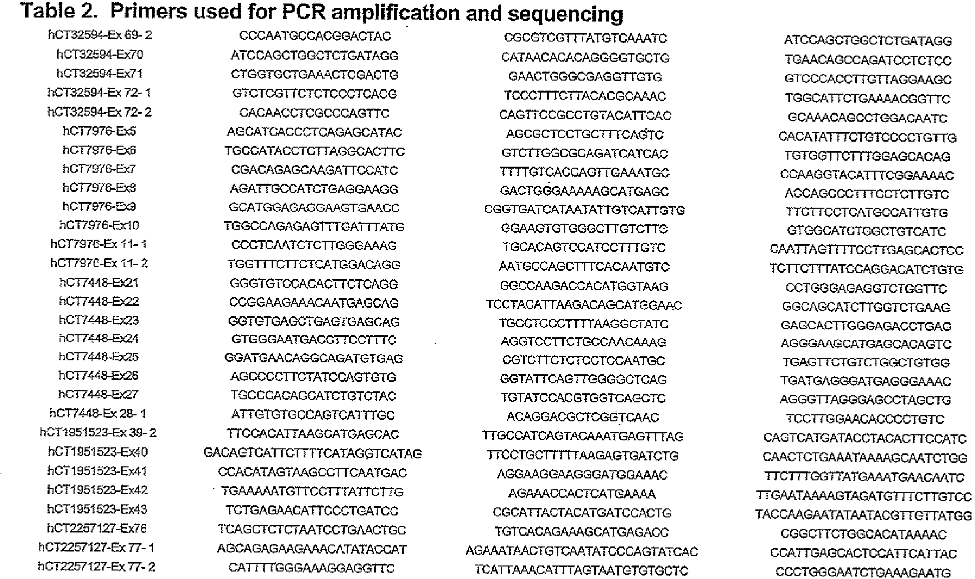

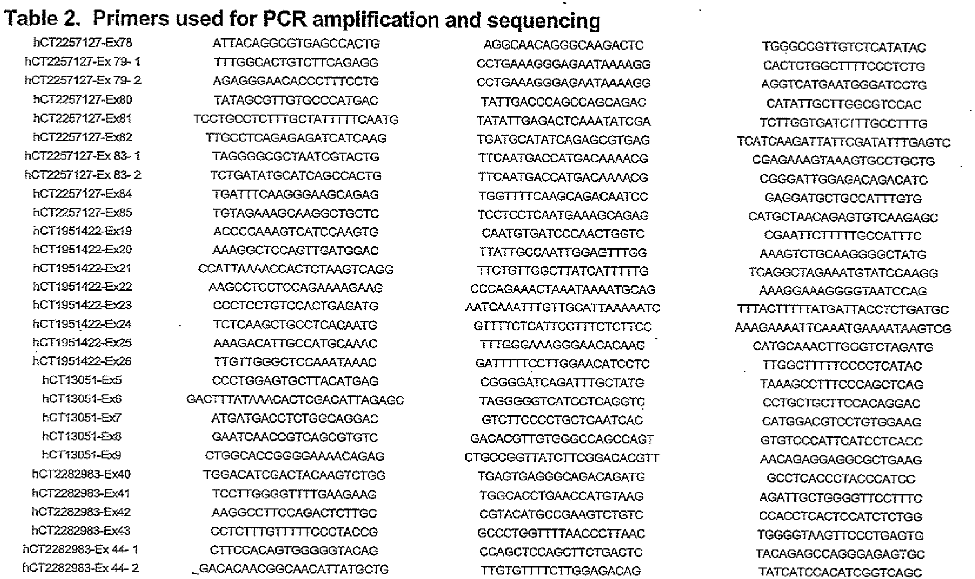

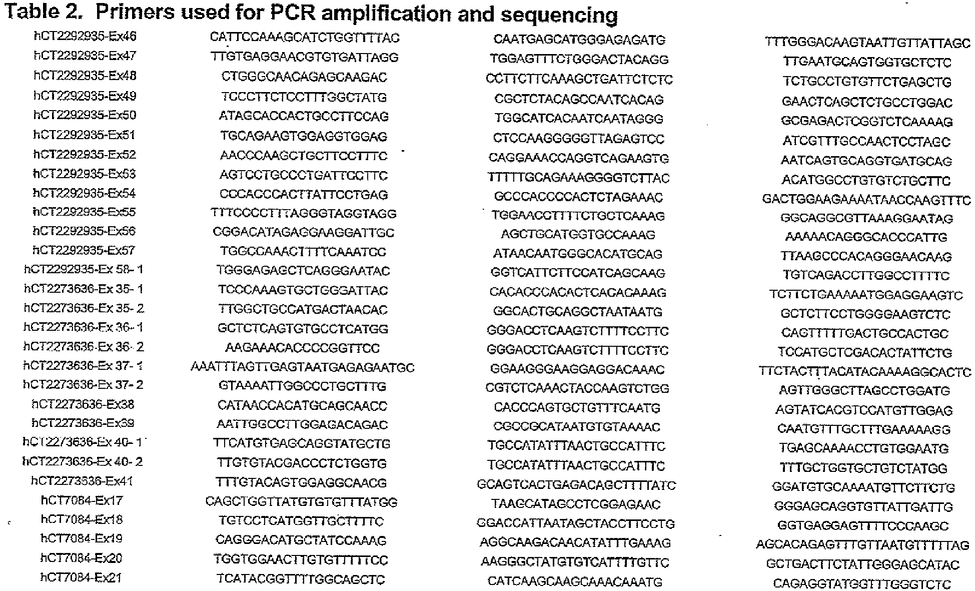

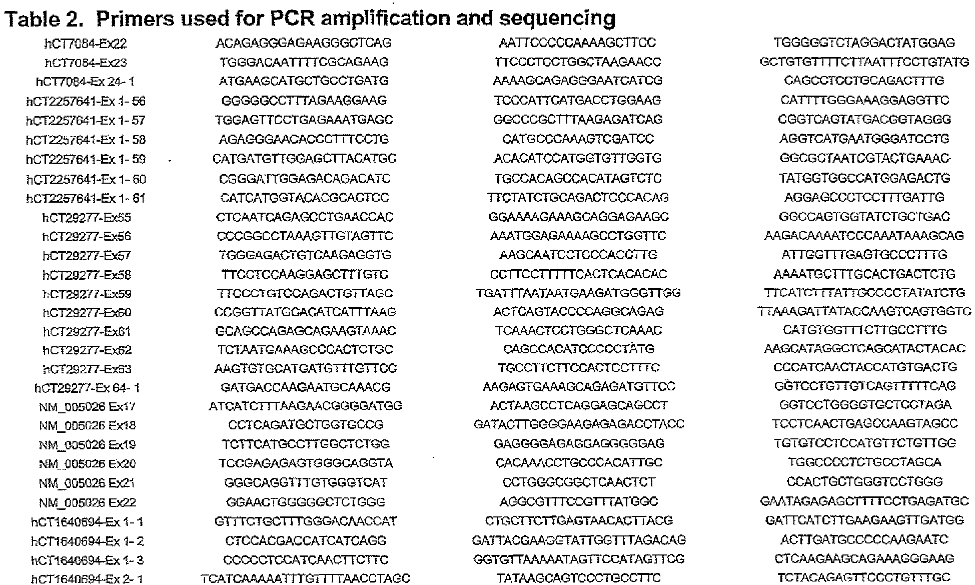

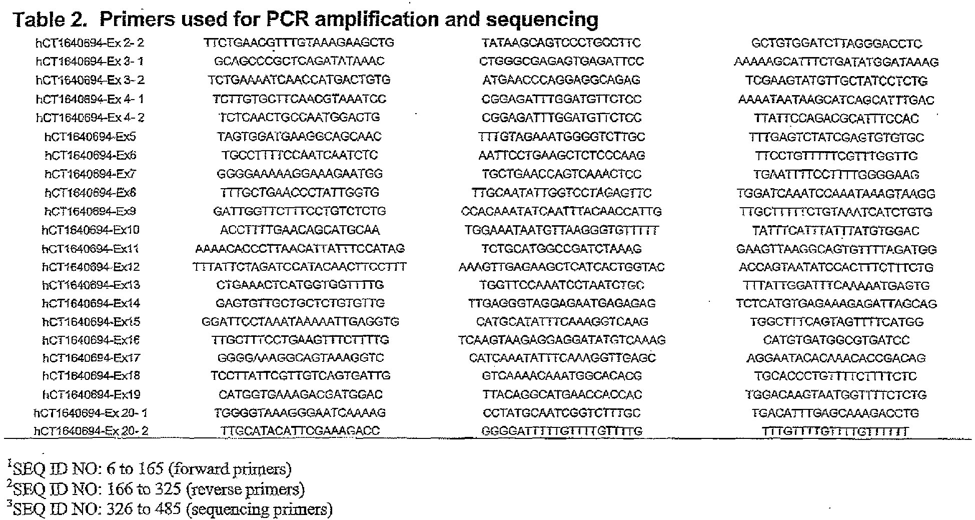

### Example 2-This example demonstrates the striking clustering of mutations within the PIK3CA gene

All coding exons of PIK3CA were then analyzed in an additional 199 colorectal cancers, revealing mutations in a total of 74 tumors (32%) (Table 3 and examples in Figure 1).

**Table 3 . PIK3CA mutations in human cancers**

| ***PIK3CA* mutations*** | | | | | **Tumor type*** | | | | | | | |
|---|---|---|---|---|---|---|---|---|---|---|---|---|
| **Exon** | **Nucleotide** | **Amino acid** | **Functional domain** | **Colon** | **GBM** | **Gastric** | **Breast** | **Lung** | **Pancreas** | **Medullo-blastomas** | **Adenomas** | **Total** |
| Exon 1 | C112T | R38C | p85 | 1 | | | | | | | | 1 |
| Exon 1 | G113A | R38H | p85 | 2 | | | | | | | | 2 |
| Exon 1 | G263A | R88Q | p85 | 1 | | | | | | | | 1 |
| Bxon 1 | C311G | P104R | p85 | 1 | | | | | | | | 1 |
| Exon 1 | G317T | G106V | p85 | 1 | | | | | | | | 1 |
| Exon 1 | G323C | R108P | p85 | 1 | | | | | | | | 1 |
| Exon 1 | del332-334 | delK111 | | 1 | | | | | | | | 1 |
| Exon 2 | G353A | G118D | | 1 | | | | | | | | 1 |
| Exon 2 | G365A | G122D | | 1 | | | | | | | | 1 |
| Exon 2 | C370A | P124T | | 1 | | | | | | | | 1 |
| Exon 4 | T1035A | N345K | C2 | 1 | | | | | | | | 1 |
| Exon 4 | G1048C | D350H | C2 | | 1 | | | | | | | 1 |
| Exon 5 | T1132C | C378R | C2 | | 1 | | | | | | | 1 |
| Exon 7 | T1258C | C420R | C2 | 2 | | | | | | | | 2 |
| Exon 7 | G1357C | E453Q | C2 | 1 | | | | | | | | 1 |
| Exon 9 | C1616G | P539R | Helical | 1 | | | | | | | | 1 |
| Exon 9 | G1624A | E542K | Helical | 9 | | | | | | | 1 | 10 |
| Exon 9 | A1625G | E542G | Helical | 1 | | | | | | | | 1 |
| Exon 9 | A1625T | E542V | Helical | | | | | | | | 1 | 1 |
| Exon 9 | G1633A | E545K | Helical | 21 | | | | 1 | | | | 22 |
| Exon 9 | A1634G | E545G | Helical | 1 | | | | | | | | 1 |
| Exon 9 | G1635T | E545D | Helical | 1 | | | | | | | | 1 |
| Exon 9 | C1636A | Q546K | Helical | 5 | | | | | | | | 5 |
| Exon 9 | A1637C | Q546P | Helical | 1 | | | | | | | | 1 |
| Exon 12 | C1981A | Q661K | Helical | 1 | | | | | | | | 1 |
| Exon 13 | A2102C | H701P | Helical | | 1 | | | | | | | 1 |
| Exon 18 | G2702T | C901F | Kinase | 1 | | 1 | | | | | | 2 |
| Exon 18 | T2725C | F909L | Kinase | 1 | | | | | | | | 1 |
| Exon 20 | T3022C | S1008P | Kinase | 1 | | | | | | | | 1 |
| Exon 20 | A3073G | T1025A | Kinase | 1 | | | | | | | | 1 |
| Exon 20 | C3074A | T1025N | Kinase | 1 | | | | | | | | 1 |
| Exon 20 | G3129T | M1043I | Kinase | 2 | | | | | | | | 2 |
| Exon 20 | C3139T | H1047Y | Kinase | 2 | | | | | | | | 2 |
| Exon 20 | A3140G | H1047R | Kinase | 15 | | 2 | 1 | | | | | 18 |
| Exon 20 | A3140T | H1047L | Kinase | 1 | | | | | | | | 1 |
| Exon 20 | G3145A | G1049S | Kinase | | 1 | | | | | | | 1 |
| Tumors with mutations | | | | 74 | 4 | 3 | 1 | 1 | 0 | 0 | 2 | |
| No. samples screened | | | | 234 | 15 | 12 | 12 | 24 | 11 | 12 | 76 | |
| Percent of tumors with mutations | | | | 32% | 27% | 25% | 8% | 4% | 0% | 0% | 3% | |

| | | | | | | | | | | | | |
|---|---|---|---|---|---|---|---|---|---|---|---|---|
| *Exon number with nucleotide and amino acid change resulting from mutation. Nucleotide position refers to position within coding sequence, where position 1 corresponds to the first poition of the start codon. Functional domains are described in Fig. 1 legend. *Number of non-synonymous mutations observed in indicated tumors. Colon, colorectal cancers; GBM. glioblastomes: gastric, gastric cancers; breast, breast cancers; lung, lung cancers; pancreas, pancreatic cancers; medulloblastomas; adenomas, benign colorectal tumors. All mutations Rated were shown to be somatic except for five colorectal cancers and one globlastoma where no corresponding normal tissue was available. Mutations were indentified in 58 of 201 mismatch repair (MMR) proficient colorectal cancers, and 16 of 33 MMR-deficient colorectal cancers. Some tumors with *PIK3CA* mutations contained mutations in *KRAS* or *BRAF* while others did not, suggesting that these genes operate through independent pathways. Seven tumors contained two somatic alterations. In addition to the 92 nonsynonymous mutations recorded in the table, we detected 3 synonymous alterations. | | | | | | | | | | | | |

### Example 3-This example demonstrates that the mutations in PIK3CA occur late in tumorigenesis.

To determine the timing of PIK3CA mutations during neoplastic progression, we evaluated 76 pre-malignant colorectal tumors of various size and degree of dysplasia. Only two PIK3CA mutations were found (E542K and E542V), both in very advanced adenomas greater than 5 cm in diameter and of tubuluvillous type. These data suggest that PIK3CA abnormalities occur at relatively late stages of neoplasia, near the time that tumors begin to invade and metastasize.

### Example 4-This example demonstrates that PIK3CA mutations in a variety of different cancer types.

We then evaluated PIK3CA for genetic alterations in other tumor types (Table 1), Mutations were identified in four of fifteen (27%) glioblastomas, three of twelve (25%) gastric cancers, one of thirteen (8%) breast, and one of twenty four (4%) lung cancers. No mutations were observed in eleven pancreatic cancers or twelve medulloblastomas. In total, 89 mutations were observed, all but 3 of which were heterozygous.

### Example 5-This example demonstrates the non-random nature of the genetic alterations observed.

The sheer number of mutations observed in PIK3CA in five different cancer types strongly suggests that these mutations are functionally important. This conclusion is buttressed by two additional independent lines of evidence. First, analysis of the ratio of non-synonymous to synonymous mutations is a good measure of selection during tumor progression, as silent alterations are unlikely to exert a growth advantage. The ratio of non-synonymous to synonymous mutations in PIK3CA was 89 to 2, far higher than the 2:1 ratio expected by chance (P<1x10⁻⁴). Second, the prevalence of non-synonymous changes located in the PI3K catalytic and accessory domains was ∼120 per Mb tumor DNA, over 100 times higher than the background mutation frequency of nonfunctional alterations observed in the genome of cancer cells (P<1x10⁻⁴) (*9*),

Although the effect of these mutations on kinase function has not yet been experimentally tested, their positions and nature within PIK3CA imply that they are likely to be activating. No truncating mutations were observed and >75% of alterations occurred in two small clusters in exons 9 and 20 (Table 2 and Figure 1). The affected residues within these clusters are highly conserved evolutionarily, retaining identity in mouse, rat, and chicken. The clustering of somatic missense mutations in specific domains is similar to that observed for activating mutations in other oncogenes, such as RAS (*10*)*,* BRAF (*11, 12*), β-catenin (*13*), and members of the tyrosine kinome (*14*).

These genetic data suggest that mutant PIK3CA is likely to function as an oncogene in human cancers.

### Example 6-This example demonstrates that gene amplification of PIK3CA is not common.

Quantitative PCR analysis of PIK3CA in 96 colorectal cancers showed no evidence of gene amplification, suggesting that gene copy alterations are not a significant mechanism of activation in this tumor type. The primers used were:
Real time PI3K hCT1640694 20-1F (intron)
TTACTTATAGGTTTCAGGAGATGTGTT (SEQ ID NO: 486); and
Real time PI3K hCT1640694 20-1R
GGGTCTTTCGAATGTATGCAATG (SEQ ID NO: 487)

The Sequence Listing appended to the end of this application contains the following sequences:
SEQ ID NO: 1=coding sequence only (nt 13 to 3201 of SEQ ID NO: 2)
SEQ ID NO: 2=mRNA sequence (NM_006218)
SEQ ID NO: 3=protein sequence (NP_P06209)
SEQ ID NO: 4=exon 9
SEQ ID NO: 5=exon 20
SEQ ID NO: 6 to 165 =forward primers
SEQ ID NO: 166 to 325=reverse primers
SEQ ID NO: 326 to 485=sequencing primers
SEQ ID NO: 486 and 487 amplification primers

### References and Notes

1. R. Katso et al., Annu Rev Cell Dev Biol 17, 615-75 (2001).
2. I. Vivanco, C. L. Sawyers, Nat Rev Cancer 2, 489-501 (Jul, 2002).
3. W. A. Phillips, F. St Clair, A. D. Munday, R. J, Thomas, C. A. Mitchel1, Cancer 83, 41-7 (Jul 1, 1998).
4. E. S. Gershtein, V. A. Shatskaya, V. D. Ermilova, N. E. Itushlinsky, M. A. Krasil'nikov, Clin Chim Acta 287, 59-67 (Sep, 1999).
5. B. Vanhaesebroeck, M. D. Waterfield, Exp Cell Res 253, 239-54 (Nov 25, 1999).
6. S. Djordjevic, P. C. Driscoll, Trends Biochem Sci 27,426-32 (Aug, 2002).
7. Catalytic subunits of PI3Ks were identified by analysis of InterPro (IPR) PI3K domains (IPR000403) present within the Celera draft human genome sequence. This resulted in identification of 15 PI3Ks and related PI3K genes. The kinase domain of PIK3CD gene was not represented in the current draft of human genome sequence and was therefore not included in this study.
8. Sequences for all annotated exons and adjacent intronic sequences containing the kinase domain of identified PI3Ks were extracted from the Celera draft human genome sequence (URL address: www host server, domain name celera.com). Celera and Genbank accession numbers of all analyzed genes are available in Table 1. Primers for PCR amplification and sequencing were designed using the Primer 3 program (URL address: http file type; www-genome.wi.mit.edu host server, cgi-bin domain name, primer directory, primer3_www.cgi subdirectory), and were synthesized by MWG (High Point, NC) or IDT (Coralville, IA). PCR amplification and sequencing were performed on tumor DNA from early passage cell lines or primary tumors as previously described (*12*) using a 384 capillary automated sequencing apparatus (Spectrumedix, State College, PA). Sequence traces were assembled and analyzed to identify potential genomic alterations using the Mutation Explorer software package (SoftGenetics, State College, PA). Of the exons extracted, 96% were successfully analyzed. Sequences of all primers used for PCR amplification and sequencing are provided in Table S1.
9. T. L. Wang et al., Proc Natl Acad Sci USA 99, 3076-80. (2002).
10. J. L. Bos et al, Nature 327, 293-7 (1987).
11. H. Davies et al., Nature (Jun 9, 2002).
12. H. Rajagopalan et al., Nature 418, 934. (2002).
13. P. J. Morin et al., Science 275, 1787-90 (1997).
14. A. Bardelli et al., Science 300, 949 (May 9, 2003).
15. J. Li et al., Science 275, 1943-7 (1997).
16. P. A. Steck et al., Nat Genet 15, 356-62 (1997).
17. T. Maehama, J. B. Dixon, J Biol Chem 273, 13375-8 (May 29, 1998).
18. M. P. Myers et al., Proc Natl Acad Sci U S A 95, 13513-8 (Nov 10,1998).
19. L. Shayesteh et al., Nat Genet 21, 99-102 (Jan, 1999).
20. J. Q. Cheng et al., Proc Natl Acad Sci U S A 89, 9267-71 (Oct 1, 1992).
21. L. Hu, J. Hofmann, Y. Lu, G. B. Mills, R. B. Jaffe, Cancer Res 62, 1087-92 (Feb 15, 2002).
22. J. Luo, B. D. Manning, L. C. Cantley, Cancer Cell 4, 257-62 (2003).

## Claims

1. A method for detecting a mutated PIK3CA polynucleotide, comprising: amplifying a PIK3CA polynucleotide; and detecting the presence of a mutation in the polynucleotide amplified in the amplification step, wherein the mutation is at least one mutation selected from the group consisting of C112T, G113A, G263A, C311G, G317T, G323C, del332-334, G353A, G365A, C370A, T1035A, G1048C, T1132C, T1258C, G1357C, C1616G, G1624A, A1625G, A1625T, G1633A, A1634G, G1635T, C1636A, A1637C, C1981A, A2102C, G2702T, T2725C, T3022C, A3073G, C3074A, G3129T, C3139T, A3140G, A3140T, G3145A in comparison with a wild type PIK3CA polynucleotide, the wild type PIK3CA polynucleotide having a nucleotide sequence of SEQ ID NO.1.

2. A method for detecting a mutated PIK3CA polynucleotide, the method comprising detecting the presence of the mutated PIK3CA polynucleotide according to claim 1 in a sample obtained from a subject.

## Patentansprüche

1. Verfahren zum Nachweis eines mutierten PIK3CA-Polynukleotids, umfassend: Amplifizieren eines PIK3CA-Polynukleotids; und Nachweisen des Vorliegens einer Mutation in dem bei dem Amplifikationsschritt amplifizierten Polynukleotid, wobei die Mutation wenigstens eine Mutation ist, die ausgewählt ist aus der Gruppe bestehend aus C112T, G113A, G263A, C311G, G317T, G323C, del332-334, G353A, G365A, C370A, T1035A, G1048C, T1132C, T1258C, G1357C, C1616G, G1624A, A1625G, A1625T, G1633A, A1634G, G1635T, C1636A, A1637C, C1981A, A2102C, G2702T, T2725C, T3022C, A3073G, C3074A, G3129T, C3139T, A3140G, A3140T, G3145A, im Vergleich mit einem Wildtyp-PIK3CA-Polynukleotid, wobei das Wildtyp-PIK3CA-Polynukleotid eine Nukleotidsequenz von SEQ ID NO.1 hat.

2. Verfahren zum Nachweis eines mutierten PIK3CA-Polynukleotids, wobei das Verfahren Nachweisen des Vorliegens des mutierten PIK3CA-Polynukleotids gemäß Anspruch 1 in einer von einer Testperson erhaltenen Probe umfasst.

## Revendications

1. Procédé pour détecter un polynucléotide PIK3CA muté, comprenant : l'amplification d'un polynucléotide PIK3CA ; et la détection de la présence d'une mutation dans le polynucléotide amplifié dans l'étape d'amplification, où la mutation est au moins une mutation choisie dans le groupe consistant en C112T, G113A, G263A, C311G, G317T, G323C, del332-334, G353A, G365A, C370A, T1035A, G1048C, T1132C, T1258C, G1357C, G1616G, G1624A, A1625G, A1625T, G1633A, A1634G, G1635T, C1636A, A1637C, C1981A, A2102C, G2702T, T2725C, T3022C, A3073G, C3074A, G3129T, C3139T, A3140G, A3140T, G3145A en comparaison avec un polynucléotide PIK3CA de type sauvage, le polynucléotide PIK3CA de type sauvage ayant une séquence nucléotidique de SEQ ID NO.1.

2. Procédé pour détecter un polynucléotide PIK3CA muté, le procédé comprenant la détection de la présence du polynucléotide PIK3CA muté selon la revendication 1 dans un échantillon obtenu auprès d'un sujet.


### SEQUENCE LISTING

<110> Velculescu, Victor
   Kinzler, Kenneth
   Vogelstein, Bert
   Samuels, Yardena
<120> MUTATIONS OF THE PIK3CA GENE IN HUMAN
   CANCERS
<130> 001107.00428
<160> 487
<170> FastSEQ for Windows Version 4.0
<210> 1
   <211> 3412
   <212> DNA
   <213> Homo sapiens
<400> 1
   atgcctccaa gaccatcatc aggtgaactg tggggcatcc acttgatgcc cccaagaatc      60
   ctagtggaat gtttactacc aaatggaatg atagtgactt tagaatgcct ccgtgaggct     120
   acattagtaa ctataaagca tgaactattt aaagaagcaa gaaaataccc tctccatcaa     180
   cttcttcaag atgaatcttc ttacattttc gtaagtgtta cccaagaagc agaaagggaa     240
   gaattttttg atgaaacaag acgactttgt gatcttcggc tttttcaacc atttttaaaa     300
   gtaattgaac cagtaggcaa ccgtgaagaa aagatcctca atcgagaaat tggttttgct     360
   atcggcatgc cagtgtgcga atttgatatg gttaaagatc ctgaagtaca ggacttccga     420
   agaaatattc ttaatgtttg taaagaagct gtggatctta gggatcttaa ttcacctcat     480
   agtagagcaa tgtatgtcta tccgccacat gtagaatctt caccagagct gccaaagcac     540
   atatataata aattggatag aggccaaata atagtggtga tttgggtaat agtttctcca     600
   aataatgaca agcagaagta tactctgaaa atcaaccatg actgtgtgcc agaacaagta     660
   attgctgaag caatcaggaa aaaaactaga agtatgttgc tatcatctga acaattaaaa     720
   ctctgtgttt tagaatatca gggcaagtac attttaaaag tgtgtggatg tgatgaatac     780
   ttcctagaaa aatatcctct gagtcagtat aagtatataa gaagctgtat aatgcttggg     840
   aggatgccca atttgaagat gatggctaaa gaaagccttt attctcaact gccaatggac     900
   tgttttacaa tgccatctta ttccagacgc atttccacag ctacaccata tatgaatgga     960
   gaaacatcta caaaatccct ttgggttata aatagagcac tcagaataaa aattctttgt    1020
   gcaacctacg tgaatctaaa tattcgagac attgacaaga tttatgttcg aacaggtatc    1080
   taccatggag gagaaccctt atgtgacaat gtgaacactc aaagagtacc ttgttccaat    1140
   cccaggtgga atgaatggct gaattatgat atatacattc ctgatcttcc tcgtgctgct    1200
   cgactttgcc tttccatttg ctctgttaaa ggccgaaagg gtgctaaaga ggaacactgt    1260
   ccattggcat ggggaaatat aaacttgttt gattacacag acactctagt atctggaaaa    1320
   atggctttga atctttggcc agtacctcat ggattagaag atttgctgaa ccctattggt    1380
   gttactggat caaatccaaa taaagaaact ccatgcttag agttggagtt tgactggttc    1440
   agcagtgtgg taaagttccc agatatgtca gtgattgaag agcatgccaa ttggtctgta    1500
   tcccgagaag caggatttag ctattcccac gcaggactga gtaacagact agctagagac    1560
   aatgaattaa gggaaaatga caaagaacag ctcaaagcaa tttctacacg agatcctctc    1620
   tctgaaatca ctgagcagga gaaagatttt ctatggagtc acagacacta ttgtgtaact    1680
   atccccgaaa ttctacccaa attgcttctg tctgttaaat ggaattctag agatgaagta    1740
   gcccagatgt attgcttggt aaaagattgg cctccaatca aacctgaaca ggctatggaa    1800
   cttctggact gtaattaccc agatcctatg gttcgaggtt ttgctgttcg gtgcttggaa    1860
   aaatatttaa cagatgacaa actttctcag tatttaattc agctagtaca ggtcctaaaa    1920
   tatgaacaat atttggataa cttgcttgtg agatttttac tgaagaaagc attgactaat    1980
   caaaggattg ggcacttttt cttttggcat ttaaaatctg agatgcacaa taaaacagtt    2040
   agccagaggt ttggcctgct tttggagtcc tattgtcgtg catgtgggat gtatttgaag    2100
   cacctgaata ggcaagtcga ggcaatggaa aagctcatta acttaactga cattctcaaa    2160
   caggagagga aggatgaaac acaaaaggta cagatgaagt ttttagttga gcaaatgagg    2220
   cgaccagatt tcatggatgc cctacagggc ttgctgtctc ctctaaaccc tgctcatcaa    2280
   ctaggaaacc tcaggcttaa agagtgtcga attatgtctt ctgcaaaaag gccactgtgg    2340
   ttgaattggg agaacccaga catcatgtca gagttactgt ttcagaacaa tgagatcatc    2400
   tttaaaaatg gggatgattt acggcaagat atgctaacac ttcaaattat tcgtattatg    2460
   gaaaatatct ggcaaaatca aggtcttgat cttcgaatgt taccttatgg ttgtctgtca    2520
   atcggtgact gtgtgggact tattgaggtg gtgcgaaatt ctcacactat tatgcaaatt    2580
   cagtgcaaag gcggcttgaa aggtgcactg cagttcaaca gccacacact acatcagtgg    2640
   ctcaaagaca agaacaaagg agaaatatat gatgcagcca ttgacctgtt tacacgttca    2700
   tgtgctggat actgtgtagc taccttcatt ttgggaattg gagatcgtca caatagtaac    2760
   atcatggtga aagacgatgg acaactgttt catatagatt ttggacactt tttggatcac    2820
   aagaagaaaa aatttggtta taaacgagaa cgtgtgccat ttgttttgac acaggatttc    2880
   ttaatagtga ttagtaaagg agcccaagaa tgcacaaaga caagagaatt tgagaggttt    2940
   caggagatgt gttacaaggc ttatctagct attcgacagc atgccaatct cttcataaat    3000
   cttttctcaa tgatgcttgg ctctggaatg ccagaactac aatcttttga tgacattgca    3060
   tacattcgaa agaccctagc cttagataaa actgagcaag aggctttgga gtatttcatg    3120
   aaacaaatga atgatgcaca tcatggtggc tggacaacaa aaatggattg gatcttccac    3180
   acaattaaac agcatgcatt gaactgaaag ataactgaga aaatgaaagc tcactctgga    3240
   ttccacactg cactgttaat aactctcagc aggcaaagac cgattgcata ggaattgcac    3300
   aatccatgaa cagcattaga tttacagcaa gaacagaaat aaaatactat ataatttaaa    3360
   taatgtaaac gcaaacaggg tttgatagca cttaaactag ttcatttcaa aa            3412
<210> 2
   <211> 3424
   <212> RNA
   <213> Homo sapiens
<400> 2
   aggatcagaa caatgcctcc aagaccatca tcaggtgaac tgtggggcat ccacttgatg      60
   cccccaagaa tcctagtgga atgtttacta ccaaatggaa tgatagtgac tttagaatgc     120
   ctccgtgagg ctacattagt aactataaag catgaactat ttaaagaagc aagaaaatac     180
   cctctccatc aacttcttca agatgaatct tcttacattt tcgtaagtgt tacccaagaa     240
   gcagaaaggg aagaattttt tgatgaaaca agacgacttt gtgatcttcg gctttttcaa     300
   ccatttttaa aagtaattga accagtaggc aaccgtgaag aaaagatcct caatcgagaa     360
   attggttttg ctatcggcat gccagtgtgc gaatttgata tggttaaaga tcctgaagta     420
   caggacttcc gaagaaatat tcttaatgtt tgtaaagaag ctgtggatct tagggatctt     480
   aattcacctc atagtagagc aatgtatgtc tatccgccac atgtagaatc ttcaccagag     540
   ctgccaaagc acatatataa taaattggat agaggccaaa taatagtggt gatttgggta     600
   atagtttctc caaataatga caagcagaag tatactctga aaatcaacca tgactgtgtg     660
   ccagaacaag taattgctga agcaatcagg aaaaaaacta gaagtatgtt gctatcatct     720
   gaacaattaa aactctgtgt tttagaatat cagggcaagt acattttaaa agtgtgtgga     780
   tgtgatgaat acttcctaga aaaatatcct ctgagtcagt ataagtatat aagaagctgt     840
   ataatgcttg ggaggatgcc caatttgaag atgatggcta aagaaagcct ttattctcaa     900
   ctgccaatgg actgttttac aatgccatct tattccagac gcatttccac agctacacca     960
   tatatgaatg gagaaacatc tacaaaatcc ctttgggtta taaatagagc actcagaata    1020
   aaaattcttt gtgcaaccta cgtgaatcta aatattcgag acattgacaa gatttatgtt    1080
   cgaacaggta tctaccatgg aggagaaccc ttatgtgaca atgtgaacac tcaaagagta    1140
   ccttgttcca atcccaggtg gaatgaatgg ctgaattatg atatatacat tcctgatctt    1200
   cctcgtgctg ctcgactttg cctttccatt tgctctgtta aaggccgaaa gggtgctaaa    1260
   gaggaacact gtccattggc atggggaaat ataaacttgt ttgattacac agacactcta    1320
   gtatctggaa aaatggcttt gaatctttgg ccagtacctc atggattaga agatttgctg    1380
   aaccctattg gtgttactgg atcaaatcca aataaagaaa ctccatgctt agagttggag    1440
   tttgactggt tcagcagtgt ggtaaagttc ccagatatgt cagtgattga agagcatgcc    1500
   aattggtctg tatcccgaga agcaggattt agctattccc acgcaggact gagtaacaga    1560
   ctagctagag acaatgaatt aagggaaaat gacaaagaac agctcaaagc aatttctaca    1620
   cgagatcctc tctctgaaat cactgagcag gagaaagatt ttctatggag tcacagacac    1680
   tattgtgtaa ctatccccga aattctaccc aaattgcttc tgtctgttaa atggaattct    1740
   agagatgaag tagcccagat gtattgcttg gtaaaagatt ggcctccaat caaacctgaa    1800
   caggctatgg aacttctgga ctgtaattac ccagatccta tggttcgagg ttttgctgtt    1860
   cggtgcttgg aaaaatattt aacagatgac aaactttctc agtatttaat tcagctagta    1920
   caggtcctaa aatatgaaca atatttggat aacttgcttg tgagattttt actgaagaaa    1980
   gcattgacta atcaaaggat tgggcacttt ttcttttggc atttaaaatc tgagatgcac    2040
   aataaaacag ttagccagag gtttggcctg cttttggagt cctattgtcg tgcatgtggg    2100
   atgtatttga agcacctgaa taggcaagtc gaggcaatgg aaaagctcat taacttaact    2160
   gacattctca aacaggagag gaaggatgaa acacaaaagg tacagatgaa gtttttagtt    2220
   gagcaaatga ggcgaccaga tttcatggat gccctacagg gcttgctgtc tcctctaaac    2280
   cctgctcatc aactaggaaa cctcaggctt aaagagtgtc gaattatgtc ttctgcaaaa    2340
   aggccactgt ggttgaattg ggagaaccca gacatcatgt cagagttact gtttcagaac    2400
   aatgagatca tctttaaaaa tggggatgat ttacggcaag atatgctaac acttcaaatt    2460
   attcgtatta tggaaaatat ctggcaaaat caaggtcttg atcttcgaat gttaccttat    2520
   ggttgtctgt caatcggtga ctgtgtggga cttattgagg tggtgcgaaa ttctcacact    2580
   attatgcaaa ttcagtgcaa aggcggcttg aaaggtgcac tgcagttcaa cagccacaca    2640
   ctacatcagt ggctcaaaga caagaacaaa ggagaaatat atgatgcagc cattgacctg    2700
   tttacacgtt catgtgctgg atactgtgta gctaccttca ttttgggaat tggagatcgt    2760
   cacaatagta acatcatggt gaaagacgat ggacaactgt ttcatataga ttttggacac    2820
   tttttggatc acaagaagaa aaaatttggt tataaacgag aacgtgtgcc atttgttttg    2880
   acacaggatt tcttaatagt gattagtaaa ggagcccaag aatgcacaaa gacaagagaa    2940
   tttgagaggt ttcaggagat gtgttacaag gcttatctag ctattcgaca gcatgccaat    3000
   ctcttcataa atcttttctc aatgatgctt ggctctggaa tgccagaact acaatctttt    3060
   gatgacattg catacattcg aaagacccta gccttagata aaactgagca agaggctttg    3120
   gagtatttca tgaaacaaat gaatgatgca catcatggtg gctggacaac aaaaatggat    3180
   tggatcttcc acacaattaa acagcatgca ttgaactgaa agataactga gaaaatgaaa    3240
   gctcactctg gattccacac tgcactgtta ataactctca gcaggcaaag accgattgca    3300
   taggaattgc acaatccatg aacagcatta gatttacagc aagaacagaa ataaaatact    3360
   atataattta aataatgtaa acgcaaacag ggtttgatag cacttaaact agttcatttc    3420
   aaaa                                                                 3424
<210> 3
   <211> 1068
   <212> PRT
   <213> Homo sapiens
<400> 3
   Met Pro Pro Arg Pro Ser Ser Gly Glu Leu Trp Gly Ile His Leu Met
    1               5                  10                  15
   Pro Pro Arg Ile Leu Val Glu Cys Leu Leu Pro Asn Gly Met Ile Val
               20                  25                  30
   Thr Leu Glu Cys Leu Arg Glu Ala Thr Leu Val Thr Ile Lys His Glu
           35                  40                  45
   Leu Phe Lys Glu Ala Arg Lys Tyr Pro Leu His Gln Leu Leu Gln Asp
       50                  55                  60
   Glu Ser Ser Tyr Ile Phe Val Ser Val Thr Gln Glu Ala Glu Arg Glu
   65                  70                  75                  80
   Glu Phe Phe Asp Glu Thr Arg Arg Leu Cys Asp Leu Arg Leu Phe Gln
                   85                  90                  95
   Pro Phe Leu Lys Val Ile Glu Pro Val Gly Asn Arg Glu Glu Lys Ile
               100                 105                 110
   Leu Asn Arg Glu Ile Gly Phe Ala Ile Gly Met Pro Val Cys Glu Phe
           115                 120                 125
   Asp Met Val Lys Asp Pro Glu Val Gln Asp Phe Arg Arg Asn Ile Leu
       130                 135                 140
   Asn Val Cys Lys Glu Ala Val Asp Leu Arg Asp Leu Asn Ser Pro His
   145                 150                 155                 160
   Ser Arg Ala Met Tyr Val Tyr Pro Pro His Val Glu Ser Ser Pro Glu
                   165                 170                 175
   Leu Pro Lys His Ile Tyr Asn Lys Leu Asp Arg Gly Gln Ile Ile Val
               180                 185                 190
   Val Ile Trp Val Ile Val Ser Pro Asn Asn Asp Lys Gln Lys Tyr Thr
           195                 200                 205
   Leu Lys Ile Asn His Asp Cys Val Pro Glu Gln Val Ile Ala Glu Ala
       210                 215                 220
   Ile Arg Lys Lys Thr Arg Ser Met Leu Leu Ser Ser Glu Gln Leu Lys
   225                 230                 235                 240
   Leu Cys Val Leu Glu Tyr Gln Gly Lys Tyr Ile Leu Lys Val Cys Gly
                   245                 250                 255
   Cys Asp Glu Tyr Phe Leu Glu Lys Tyr Pro Leu Ser Gln Tyr Lys Tyr
               260                 265                 270
   Ile Arg Ser Cys Ile Met Leu Gly Arg Met Pro Asn Leu Lys Met Met
           275                 280                 285
   Ala Lys Glu Ser Leu Tyr Ser Gln Leu Pro Met Asp Cys Phe Thr Met
       290                 295                 300
   Pro Ser Tyr Ser Arg Arg Ile Ser Thr Ala Thr Pro Tyr Met Asn Gly
   305                 310                 315                 320
   Glu Thr Ser Thr Lys Ser Leu Trp Val Ile Asn Arg Ala Leu Arg Ile
                   325                 330                 335
   Lys Ile Leu Cys Ala Thr Tyr Val Asn Leu Asn Ile Arg Asp Ile Asp
               340                 345                 350
   Lys Ile Tyr Val Arg Thr Gly Ile Tyr His Gly Gly Glu Pro Leu Cys
           355                 360                 365
   Asp Asn Val Asn Thr Gln Arg Val Pro Cys Ser Asn Pro Arg Trp Asn
       370                 375                 380
   Glu Trp Leu Asn Tyr Asp Ile Tyr Ile Pro Asp Leu Pro Arg Ala Ala
   385                 390                 395                 400
   Arg Leu Cys Leu Ser Ile Cys Ser Val Lys Gly Arg Lys Gly Ala Lys
                   405                 410                 415
   Glu Glu His Cys Pro Leu Ala Trp Gly Asn Ile Asn Leu Phe Asp Tyr
               420                 425                 430
   Thr Asp Thr Leu Val Ser Gly Lys Met Ala Leu Asn Leu Trp Pro Val
           435                 440                 445
   Pro His Gly Leu Glu Asp Leu Leu Asn Pro Ile Gly Val Thr Gly Ser
       450                 455                 460
   Asn Pro Asn Lys Glu Thr Pro Cys Leu Glu Leu Glu Phe Asp Trp Phe
   465                 470                 475                 480
   Ser Ser Val Val Lys Phe Pro Asp Met Ser Val Ile Glu Glu His Ala
                   485                 490                 495
   Asn Trp Ser Val Ser Arg Glu Ala Gly Phe Ser Tyr Ser His Ala Gly
               500                 505                 510
   Leu Ser Asn Arg Leu Ala Arg Asp Asn Glu Leu Arg Glu Asn Asp Lys
           515                 520                 525
   Glu Gln Leu Lys Ala Ile Ser Thr Arg Asp Pro Leu Ser Glu Ile Thr
       530                 535                 540
   Glu Gln Glu Lys Asp Phe Leu Trp Ser His Arg His Tyr Cys Val Thr
   545                 550                 555                 560
   Ile Pro Glu Ile Leu Pro Lys Leu Leu Leu Ser Val Lys Trp Asn Ser
                   565                 570                 575
   Arg Asp Glu Val Ala Gln Met Tyr Cys Leu Val Lys Asp Trp Pro Pro
               580                 585                 590
   Ile Lys Pro Glu Gln Ala Met Glu Leu Leu Asp Cys Asn Tyr Pro Asp
           595                 600                 605
   Pro Met Val Arg Gly Phe Ala Val Arg Cys Leu Glu Lys Tyr Leu Thr
       610                 615                 620
   Asp Asp Lys Leu Ser Gln Tyr Leu Ile Gln Leu Val Gln Val Leu Lys
   625                 630                 635                 640
   Tyr Glu Gln Tyr Leu Asp Asn Leu Leu Val Arg Phe Leu Leu Lys Lys
                   645                 650                 655
   Ala Leu Thr Asn Gln Arg Ile Gly His Phe Phe Phe Trp His Leu Lys
               660                 665                 670
   Ser Glu Met His Asn Lys Thr Val Ser Gln Arg Phe Gly Leu Leu Leu
           675                 680                 685
   Glu Ser Tyr Cys Arg Ala Cys Gly Met Tyr Leu Lys His Leu Asn Arg
       690                 695                 700
   Gln Val Glu Ala Met Glu Lys Leu Ile Asn Leu Thr Asp Ile Leu Lys
   705                 710                 715                 720
   Gln Glu Arg Lys Asp Glu Thr Gln Lys Val Gln Met Lys Phe Leu Val
                   725                 730                 735
   Glu Gln Met Arg Arg Pro Asp Phe Met Asp Ala Leu Gln Gly Leu Leu
               740                 745                 750
   Ser Pro Leu Asn Pro Ala His Gln Leu Gly Asn Leu Arg Leu Lys Glu
           755                 760                 765
   Cys Arg Ile Met Ser Ser Ala Lys Arg Pro Leu Trp Leu Asn Trp Glu
       770                 775                 780
   Asn Pro Asp Ile Met Ser Glu Leu Leu Phe Gln Asn Asn Glu Ile Ile
   785                 790                 795                 800
   Phe Lys Asn Gly Asp Asp Leu Arg Gln Asp Met Leu Thr Leu Gln Ile
                   805                 810                 815
   Ile Arg Ile Met Glu Asn Ile Trp Gln Asn Gln Gly Leu Asp Leu Arg
               820                 825                 830
   Met Leu Pro Tyr Gly Cys Leu Ser Ile Gly Asp Cys Val Gly Leu Ile
           835                 840                 845
   Glu Val Val Arg Asn Ser His Thr Ile Met Gln Ile Gln Cys Lys Gly
       850                 855                 860
   Gly Leu Lys Gly Ala Leu Gln Phe Asn Ser His Thr Leu His Gln Trp
   865                 870                 875                 880
   Leu Lys Asp Lys Asn Lys Gly Glu Ile Tyr Asp Ala Ala Ile Asp Leu
                   885                 890                 895
   Phe Thr Arg Ser Cys Ala Gly Tyr Cys Val Ala Thr Phe Ile Leu Gly
               900                 905                 910
   Ile Gly Asp Arg His Asn Ser Asn Ile Met Val Lys Asp Asp Gly Gln
           915                 920                 925
   Leu Phe His Ile Asp Phe Gly His Phe Leu Asp His Lys Lys Lys Lys
       930                 935                 940
   Phe Gly Tyr Lys Arg Glu Arg Val Pro Phe Val Leu Thr Gln Asp Phe
   945                 950                 955                 960
   Leu Ile Val Ile Ser Lys Gly Ala Gln Glu Cys Thr Lys Thr Arg Glu
                   965                 970                 975
   Phe Glu Arg Phe Gln Glu Met Cys Tyr Lys Ala Tyr Leu Ala Ile Arg
               980                 985                 990
   Gln His Ala Asn Leu Phe Ile Asn Leu Phe Ser Met Met Leu Gly Ser
           995                 1000                1005
   Gly Met Pro Glu Leu Gln Ser Phe Asp Asp Ile Ala Tyr Ile Arg Lys
       1010                1015                1020
   Thr Leu Ala Leu Asp Lys Thr Glu Gln Glu Ala Leu Glu Tyr Phe Met
   1025                1030                1035                1040
   Lys Gln Met Asn Asp Ala His His Gly Gly Trp Thr Thr Lys Met Asp
                   1045                1050                1055
   Trp Ile Phe His Thr Ile Lys Gln His Ala Leu Asn
               1060                1065
<210> 4
   <211> 125
   <212> DNA
   <213> Homo sapiens
<400> 4
   agtaacagac tagctagaga caatgaatta agggaaaatg acaaagaaca gctcaaagca      60
   atttctacac gagatcctct ctctgaaatc actgagcagg agaaagattt tctatggagt     120
   cacag                                                                 125
<210> 5
   <211> 1186
   <212> DNA
   <213> Homo sapiens
<400> 5
   gtttcaggag atgtgttaca aggcttatct agctattcga cagcatgcca atctcttcat      60
   aaatcttttc tcaatgatgc ttggctctgg aatgccagaa ctacaatctt ttgatgacat     120
   tgcatacatt cgaaagaccc tagccttaga taaaactgag caagaggctt tggagtattt     180
   catgaaacaa atgaatgatg cacatcatgg tggctggaca acaaaaatgg attggatctt     240
   ccacacaatt aaacagcatg cattgaactg aaaagataac tgagaaaatg aaagctcact     300
   ctggattcca cactgcactg ttaataactc tcagcaggca aagaccgatt gcataggaat     360
   tgcacaatcc atgaacagca ttagaattta cagcaagaac agaaataaaa tactatataa     420
   tttaaataat gtaaacgcaa acagggtttg atagcactta aactagttca tttcaaaatt     480
   aagctttaga ataatgcgca atttcatgtt atgccttaag tccaaaaagg taaactttga     540
   agattgtttg tatctttttt taaaaaacaa aacaaaacaa aaatccccaa aatatataga     600
   aatgatggag aaggaaaaag tgatggtttt ttttgtcttg caaatgttct atgttttgaa     660
   atgtggacac aacaaaggct gttattgcat taggtgtaag taaactggag tttatgttaa     720
   attacattga ttggaaaaga atgaaaattt cttatttttc cattgctgtt caatttatag     780
   tttgaagtgg gtttttgact gcttgtttaa tgaagaaaaa tgcttggggt ggaagggact     840
   cttgagattt caccagagac tttttctttt taataaatca aacctttgga tgatttgagg      900
   ttttatctgc agttttggaa gcagtcacaa atgagacctg ttataaggtg gtattttttt     960
   ttttcttctg gacagtattt aaaggatctt attcttattt cccagggaaa ttctgggctc    1020
   ccacaaagta aaaaaaaaaa aaaatcatag aaaaagaatg agcaggaata gttcttattc    1080
   cagaattgta cagtattcac cttaagttga ttttttttct ccttctgcaa ttgaactgaa    1140
   tacatttttc atgcatgttt tccagaaaat agaagtatta atgtta                   1186
<210> 6
   <211> 20
   <212> DNA
   <213> Homo sapiens
<400> 6
   ttccagcctg ggtaacaaag 20
<210> 7
   <211> 20
   <212> DNA
   <213> Homo sapiens
<400> 7
   cctgacctca ggtgttctgc 20
<210> 8
   <211> 21
   <212> DNA
   <213> Homo sapiens
<400> 8
   tgcacattct gcacgtgtat c 21
<210> 9
   <211> 24
   <212> DNA
   <213> Homo sapiens
<400> 9
   tcccagtttg tatgctattg agag 24
<210> 10
   <211> 21
   <212> DNA
   <213> Homo sapiens
<400> 10
   tggaaattca aaagtgtgtg g 21
<210> 11
   <211> 23
   <212> DNA
   <213> Homo sapiens
<400> 11
   cactaatgaa cccctcaaga ctg 23
<210> 12
   <211> 20
   <212> DNA
   <213> Homo sapiens
<400> 12
   tccttggcaa agtgacaatc 20
<210> 13
   <211> 23
   <212> DNA
   <213> Homo sapiens
<400> 13
   ctctcacata caacaccatc tcc 23
<210> 14
   <211> 23
   <212> DNA
   <213> Homo sapiens
<400> 14
   atgtatctca ttgaaaaccc aac 23
<210> 15
   <211> 20
   <212> DNA
   <213> Homo sapiens
<400> 15
   tcccaaagtg ctgggattac 20
<210> 16
   <211> 26
   <212> DNA
   <213> Homo sapiens
<400> 16
   cctatgacat aaatgccagt acaaac 26
<210> 17
   <211> 24
   <212> DNA
   <213> Homo sapiens
<400> 17
   tcttttgttc agtcagcatc tctc 24
<210> 18
   <211> 24
   <212> DNA
   <213> Homo sapiens
<400> 18
   ttgagaattc agatgagaaa ccag 24
<210> 19
   <211> 20
   <212> DNA
   <213> Homo sapiens
<400> 19
   gaaggccact ctcaaacctg 20
<210> 20
   <211> 20
   <212> DNA
   <213> Homo sapiens
<400> 20
   tcaaggcttg catttcattg 20
<210> 21
   <211> 20
   <212> DNA
   <213> Homo sapiens
<400> 21
   ttccacactc caaagaatgc 20
<210> 22
   <211> 21
   <212> DNA
   <213> Homo sapiens
<400> 22
   aattgcaatc ctcttggtag c 21
<210> 23
   <211> 19
   <212> DNA
   <213> Homo sapiens
<400> 23
   gccaagacca agcaactcc 19
<210> 24
   <211> 20
   <212> DNA
   <213> Homo sapiens
<400> 24
   ataaacgacc gctggcctac 20
<210> 25
   <211> 20
   <212> DNA
   <213> Homo sapiens
<400> 25
   gtacatccgg ggacacaatg 20
<210> 26
   <211> 20
   <212> DNA
   <213> Homo sapiens
<400> 26
   accgggttct tccagctaag 20
<210> 27
   <211> 20
   <212> DNA
   <213> Homo sapiens
<400> 27
   caatgcgtgc gttaaatctg 20
<210> 28
   <211> 18
   <212> DNA
   <213> Homo sapiens
<400> 28
   cccaatgcca cggactac 18
<210> 29
   <211> 20
   <212> DNA
   <213> Homo sapiens
<400> 29
   atccagctgg ctctgatagg 20
<210> 30
   <211> 20
   <212> DNA
   <213> Homo sapiens
<400> 30
   ctggtgctga aactcgactg 20
<210> 31
   <211> 20
   <212> DNA
   <213> Homo sapiens
<400> 31
   gtctcgttct ctccctcacg 20
<210> 32
   <211> 18
   <212> DNA
   <213> Homo sapiens
<400> 32
   cacaacctcg cccagttc 18
<210> 33
   <211> 21
   <212> DNA
   <213> Homo sapiens
<400> 33
   agcatcaccc tcagagcata c 21
<210> 34
   <211> 22
   <212> DNA
   <213> Homo sapiens
<400> 34
   tgccatacct cttaggcact tc 22
<210> 35
   <211> 20
   <212> DNA
   <213> Homo sapiens
<400> 35
   cgacagagca agattccatc 20
<210> 36
   <211> 20
   <212> DNA
   <213> Homo sapiens
<400> 36
   agattgccat ctgaggaagg 20
<210> 37
   <211> 20
   <212> DNA
   <213> Homo sapiens
<400> 37
   gcatggagag gaagtgaacc 20
<210> 38
   <211> 22
   <212> DNA
   <213> Homo sapiens
<400> 38
   tggccagaga gtttgattta tg 22
<210> 39
   <211> 20
   <212> DNA
   <213> Homo sapiens
<400> 39
   ccctcaatct cttgggaaag 20
<210> 40
   <211> 21
   <212> DNA
   <213> Homo sapiens
<400> 40
   tggtttcttc tcatggacag g 21
<210> 41
   <211> 20
   <212> DNA
   <213> Homo sapiens
<400> 41
   gggtgtccac acttctcagg 20
<210> 42
   <211> 20
   <212> DNA
   <213> Homo sapiens
<400> 42
   ccggaagaaa caatgagcag 20
<210> 43
   <211> 20
   <212> DNA
   <213> Homo sapiens
<400> 43
   ggtgtgagct gagtgagcag 20
<210> 44
   <211> 20
   <212> DNA
   <213> Homo sapiens
<400> 44
   gtgggaatga ccttcctttc 20
<210> 45
   <211> 21
   <212> DNA
   <213> Homo sapiens
<400> 45
   ggatgaacag gcagatgtga g 21
<210> 46
   <211> 20
   <212> DNA
   <213> Homo sapiens
<400> 46
   agccccttct atccagtgtg 20
<210> 47
   <211> 20
   <212> DNA
   <213> Homo sapiens
<400> 47
   tgcccacagc atctgtctac 20
<210> 48
   <211> 20
   <212> DNA
   <213> Homo sapiens
<400> 48
   attgtgtgcc agtcatttgc 20
<210> 49
   <211> 21
   <212> DNA
   <213> Homo sapiens
<400> 49
   ttccacatta agcatgagca c 21
<210> 50
   <211> 27
   <212> DNA
   <213> Homo sapiens
<400> 50
   gacagtcatt cttttcatag gtcatag 27
<210> 51
   <211> 23
   <212> DNA
   <213> Homo sapiens
<400> 51
   ccacatagta agccttcaat gac 23
<210> 52
   <211> 23
   <212> DNA
   <213> Homo sapiens
<400> 52
   tgaaaaatgt tcctttattc ttg 23
<210> 53
   <211> 21
   <212> DNA
   <213> Homo sapiens
<400> 53
   tctgagaaca ttccctgatc c 21
<210> 54
   <211> 23
   <212> DNA
   <213> Homo sapiens
<400> 54
   tcagctctct aatcctgaac tgc 23
<210> 55
   <211> 23
   <212> DNA
   <213> Homo sapiens
<400> 55
   agcagagaag aaacatatac cat 23
<210> 56
   <211> 20
   <212> DNA
   <213> Homo sapiens
<400> 56
   cattttggga aaggaggttc 20
<210> 57
   <211> 20
   <212> DNA
   <213> Homo sapiens
<400> 57
   attacaggcg tgagccactg 20
<210> 58
   <211> 20
   <212> DNA
   <213> Homo sapiens
<400> 58
   tttggcactg tcttcagagg 20
<210> 59
   <211> 20
   <212> DNA
   <213> Homo sapiens
<400> 59
   agagggaaca ccctttcctg 20
<210> 60
   <211> 20
   <212> DNA
   <213> Homo sapiens
<400> 60
   tatagcgttg tgcccatgac 20
<210> 61
   <211> 26
   <212> DNA
   <213> Homo sapiens
<400> 61
   tcctgcctct ttgctatttt tcaatg 26
<210> 62
   <211> 22
   <212> DNA
   <213> Homo sapiens
<400> 62
   ttgcctcaga gagatcatca ag 22
<210> 63
   <211> 20
   <212> DNA
   <213> Homo sapiens
<400> 63
   taggggcgct aatcgtactg 20
<210> 64
   <211> 21
   <212> DNA
   <213> Homo sapiens
<400> 64
   tctgatatgc atcagccact g 21
<210> 65
   <211> 20
   <212> DNA
   <213> Homo sapiens
<400> 65
   tgatttcaag ggaagcagag 20
<210> 66
   <211> 20
   <212> DNA
   <213> Homo sapiens
<400> 66
   tgtagaaagc aaggctgctc 20
<210> 67
   <211> 20
   <212> DNA
   <213> Homo sapiens
<400> 67
   accccaaagt catccaagtg 20
<210> 68
   <211> 20
   <212> DNA
   <213> Homo sapiens
<400> 68
   aaaggctcca gttgatggac 20
<210> 69
   <211> 24
   <212> DNA
   <213> Homo sapiens
<400> 69
   ccattaaaac cactctaagt cagg 24
<210> 70
   <211> 21
   <212> DNA
   <213> Homo sapiens
<400> 70
   aagcctcctc cagaaaagaa g 21
<210> 71
   <211> 20
   <212> DNA
   <213> Homo sapiens
<400> 71
   ccctcctgtc cactgagatg 20
<210> 72
   <211> 20
   <212> DNA
   <213> Homo sapiens
<400> 72
   tctcaagctg cctcacaatg 20
<210> 73
   <211> 20
   <212> DNA
   <213> Homo sapiens
<400> 73
   aaagacattg ccatgcaaac 20
<210> 74
   <211> 20
   <212> DNA
   <213> Homo sapiens
<400> 74
   ttgttgggct ccaaataaac 20
<210> 75
   <211> 20
   <212> DNA
   <213> Homo sapiens
<400> 75
   ccctggagtg cttacatgag 20
<210> 76
   <211> 27
   <212> DNA
   <213> Homo sapiens
<400> 76
   gactttataa acactcgaca ttagagc 27
<210> 77
   <211> 20
   <212> DNA
   <213> Homo sapiens
<400> 77
   atgatgacct ctggcaggac 20
<210> 78
   <211> 20
   <212> DNA
   <213> Homo sapiens
<400> 78
   gaatcaaccg tcagcgtgtc 20
<210> 79
   <211> 21
   <212> DNA
   <213> Homo sapiens
<400> 79
   ctggcaccgg ggaaaacaga g 21
<210> 80
   <211> 22
   <212> DNA
   <213> Homo sapiens
<400> 80
   tggacatcga ctacaagtct gg 22
<210> 81
   <211> 20
   <212> DNA
   <213> Homo sapiens
<400> 81
   tccttggggt tttgaagaag 20
<210> 82
   <211> 20
   <212> DNA
   <213> Homo sapiens
<400> 82
   aaggccttcc agactcttgc 20
<210> 83
   <211> 21
   <212> DNA
   <213> Homo sapiens
<400> 83
   cctctttgtt tttccctacc g 21
<210> 84
   <211> 20
   <212> DNA
   <213> Homo sapiens
<400> 84
   cttccacagt gggggtacag 20
<210> 85
   <211> 23
   <212> DNA
   <213> Homo sapiens
<400> 85
   gacacaacgg caacattatg ctg 23
<210> 86
   <211> 23
   <212> DNA
   <213> Homo sapiens
<400> 86
   cattccaaag catctggttt tac 23
<210> 87
   <211> 22
   <212> DNA
   <213> Homo sapiens
<400> 87
   ttgtgaggaa cgtgtgatta gg 22
<210> 88
   <211> 19
   <212> DNA
   <213> Homo sapiens
<400> 88
   ctgggcaaca gagcaagac 19
<210> 89
   <211> 20
   <212> DNA
   <213> Homo sapiens
<400> 89
   tcccttctcc tttggctatg 20
<210> 90
   <211> 20
   <212> DNA
   <213> Homo sapiens
<400> 90
   atagcaccac tgccttccag 20
<210> 91
   <211> 19
   <212> DNA
   <213> Homo sapiens
<400> 91
   tgcagaagtg gaggtggag 19
<210> 92
   <211> 20
   <212> DNA
   <213> Homo sapiens
<400> 92
   aacccaagct gcttcctttc 20
<210> 93
   <211> 20
   <212> DNA
   <213> Homo sapiens
<400> 93
   agtcctgccc tgattccttc 20
<210> 94
   <211> 20
   <212> DNA
   <213> Homo sapiens
<400> 94
   cccacccact tattcctgag 20
<210> 95
   <211> 22
   <212> DNA
   <213> Homo sapiens
<400> 95
   tttccccttt agggtaggta gg 22
<210> 96
   <211> 21
   <212> DNA
   <213> Homo sapiens
<400> 96
   cggacataga ggaaggattg c 21
<210> 97
   <211> 20
   <212> DNA
   <213> Homo sapiens
<400> 97
   tggccaaact tttcaaatcc 20
<210> 98
   <211> 20
   <212> DNA
   <213> Homo sapiens
<400> 98
   tgggagagct cagggaatac 20
<210> 99
   <211> 20
   <212> DNA
   <213> Homo sapiens
<400> 99
   tcccaaagtg ctgggattac 20
<210> 100
   <211> 20
   <212> DNA
   <213> Homo sapiens
<400> 100
   ttggctgcca tgactaacac 20
<210> 101
   <211> 20
   <212> DNA
   <213> Homo sapiens
<400> 101
   gctctcagtg tgcctcatgg 20
<210> 102
   <211> 18
   <212> DNA
   <213> Homo sapiens
<400> 102
   aagaaacacc ccggttcc 18
<210> 103
   <211> 27
   <212> DNA
   <213> Homo sapiens
<400> 103
   aaatttagtt gagtaatgag agaatgc 27
<210> 104
   <211> 20
   <212> DNA
   <213> Homo sapiens
<400> 104
   gtaaaattgg ccctgctttg 20
<210> 105
   <211> 20
   <212> DNA
   <213> Homo sapiens
<400> 105
   cataaccaca tgcagcaacc 20
<210> 106
   <211> 20
   <212> DNA
   <213> Homo sapiens
<400> 106
   aattggcctt ggagacagac 20
<210> 107
   <211> 21
   <212> DNA
   <213> Homo sapiens
<400> 107
   ttcatgtgag caggtatgct g 21
<210> 108
   <211> 20
   <212> DNA
   <213> Homo sapiens
<400> 108
   ttgtgtacga ccctctggtg 20
<210> 109
   <211> 20
   <212> DNA
   <213> Homo sapiens
<400> 109
   tttgtacagt ggaggcaacg 20
<210> 110
   <211> 23
   <212> DNA
   <213> Homo sapiens
<400> 110
   cagctggtta tgtgtgttta tgg 23
<210> 111
   <211> 20
   <212> DNA
   <213> Homo sapiens
<400> 111
   tgtcctcatg gttgcttttc 20
<210> 112
   <211> 20
   <212> DNA
   <213> Homo sapiens
<400> 112
   cagggacatg ctatccaaag 20
<210> 113
   <211> 21
   <212> DNA
   <213> Homo sapiens
<400> 113
   tggtggaact tgtgtttttc c 21
<210> 114
   <211> 20
   <212> DNA
   <213> Homo sapiens
<400> 114
   tcatacggtt ttggcagctc 20
<210> 115
   <211> 20
   <212> DNA
   <213> Homo sapiens
<400> 115
   acagagggag aagggctcag 20
<210> 116
   <211> 20
   <212> DNA
   <213> Homo sapiens
<400> 116
   tgggacaatt ttcgcagaag 20
<210> 117
   <211> 20
   <212> DNA
   <213> Homo sapiens
<400> 117
   atgaagcatg ctgcctgatg 20
<210> 118
   <211> 19
   <212> DNA
   <213> Homo sapiens
<400> 118
   gggggccttt agaaggaag 19
<210> 119
   <211> 21
   <212> DNA
   <213> Homo sapiens
<400> 119
   tggagttcct gagaaatgag c 21
<210> 120
   <211> 20
   <212> DNA
   <213> Homo sapiens
<400> 120
   agagggaaca ccctttcctg 20
<210> 121
   <211> 22
   <212> DNA
   <213> Homo sapiens
<400> 121
   catgatgttg gagcttacat gc 22
<210> 122
   <211> 20
   <212> DNA
   <213> Homo sapiens
<400> 122
   cgggattgga gacagacatc 20
<210> 123
   <211> 20
   <212> DNA
   <213> Homo sapiens
<400> 123
   catcatggta cacgcactcc 20
<210> 124
   <211> 21
   <212> DNA
   <213> Homo sapiens
<400> 124
   ctcaatcaga gcctgaacca c 21
<210> 125
   <211> 21
   <212> DNA
   <213> Homo sapiens
<400> 125
   cccggcctaa agttgtagtt c 21
<210> 126
   <211> 20
   <212> DNA
   <213> Homo sapiens
<400> 126
   tgggagactg tcaagaggtg 20
<210> 127
   <211> 20
   <212> DNA
   <213> Homo sapiens
<400> 127
   ttcctccaag gagctttgtc 20
<210> 128
   <211> 21
   <212> DNA
   <213> Homo sapiens
<400> 128
   ttccctgtcc agactgttag c 21
<210> 129
   <211> 22
   <212> DNA
   <213> Homo sapiens
<400> 129
   ccggttatgc acatcattta ag 22
<210> 130
   <211> 21
   <212> DNA
   <213> Homo sapiens
<400> 130
   gcagccagag cagaagtaaa c 21
<210> 131
   <211> 21
   <212> DNA
   <213> Homo sapiens
<400> 131
   tctaatgaaa gcccactctg c 21
<210> 132
   <211> 22
   <212> DNA
   <213> Homo sapiens
<400> 132
   aagtgtgcat gatgtttgtt cc 22
<210> 133
   <211> 20
   <212> DNA
   <213> Homo sapiens
<400> 133
   gatgaccaag aatgcaaacg 20
<210> 134
   <211> 23
   <212> DNA
   <213> Homo sapiens
<400> 134
   atcatcttta agaacgggga tgg 23
<210> 135
   <211> 18
   <212> DNA
   <213> Homo sapiens
<400> 135
   cctcagatgc tggtgccg 18
<210> 136
   <211> 20
   <212> DNA
   <213> Homo sapiens
<400> 136
   tcttcatgcc ttggctctgg 20
<210> 137
   <211> 20
   <212> DNA
   <213> Homo sapiens
<400> 137
   tccgagagag tgggcaggta 20
<210> 138
   <211> 19
   <212> DNA
   <213> Homo sapiens
<400> 138
   gggcaggttt gtgggtcat 19
<210> 139
   <211> 18
   <212> DNA
   <213> Homo sapiens
<400> 139
   ggaactgggg gctctggg 18
<210> 140
   <211> 22
   <212> DNA
   <213> Homo sapiens
<400> 140
   gtttctgctt tgggacaacc at 22
<210> 141
   <211> 19
   <212> DNA
   <213> Homo sapiens
<400> 141
   ctccacgacc atcatcagg 19
<210> 142
   <211> 20
   <212> DNA
   <213> Homo sapiens
<400> 142
   ccccctccat caacttcttc 20
<210> 143
   <211> 26
   <212> DNA
   <213> Homo sapiens
<400> 143
   tcatcaaaaa tttgttttaa cctagc 26
<210> 144
   <211> 24
   <212> DNA
   <213> Homo sapiens
<400> 144
   ttctgaacgt ttgtaaagaa gctg 24
<210> 145
   <211> 21
   <212> DNA
   <213> Homo sapiens
<400> 145
   gcagcccgct cagatataaa c 21
<210> 146
   <211> 23
   <212> DNA
   <213> Homo sapiens
<400> 146
   tctgaaaatc aaccatgact gtg 23
<210> 147
   <211> 22
   <212> DNA
   <213> Homo sapiens
<400> 147
   tcttgtgctt caacgtaaat cc 22
<210> 148
   <211> 20
   <212> DNA
   <213> Homo sapiens
<400> 148
   tctcaactgc caatggactg 20
<210> 149
   <211> 20
   <212> DNA
   <213> Homo sapiens
<400> 149
   tagtggatga aggcagcaac 20
<210> 150
   <211> 20
   <212> DNA
   <213> Homo sapiens
<400> 150
   tgccttttcc aatcaatctc 20
<210> 151
   <211> 20
   <212> DNA
   <213> Homo sapiens
<400> 151
   ggggaaaaag gaaagaatgg 20
<210> 152
   <211> 20
   <212> DNA
   <213> Homo sapiens
<400> 152
   tttgctgaac cctattggtg 20
<210> 153
   <211> 22
   <212> DNA
   <213> Homo sapiens
<400> 153
   gattggttct ttcctgtctc tg 22
<210> 154
   <211> 20
   <212> DNA
   <213> Homo sapiens
<400> 154
   accttttgaa cagcatgcaa 20
<210> 155
   <211> 27
   <212> DNA
   <213> Homo sapiens
<400> 155
   aaaacaccct taacattatt tccatag 27
<210> 156
   <211> 28
   <212> DNA
   <213> Homo sapiens
<400> 156
   tttattctag atccatacaa cttccttt 28
<210> 157
   <211> 21
   <212> DNA
   <213> Homo sapiens
<400> 157
   ctgaaactca tggtggtttt g 21
<210> 158
   <211> 21
   <212> DNA
   <213> Homo sapiens
<400> 158
   gagtgttgct gctctgtgtt g 21
<210> 159
   <211> 25
   <212> DNA
   <213> Homo sapiens
<400> 159
   ggattcctaa ataaaaattg aggtg 25
<210> 160
   <211> 23
   <212> DNA
   <213> Homo sapiens
<400> 160
   ttgctttcct gaagtttctt ttg 23
<210> 161
   <211> 20
   <212> DNA
   <213> Homo sapiens
<400> 161
   ggggaaaggc agtaaaggtc 20
<210> 162
   <211> 23
   <212> DNA
   <213> Homo sapiens
<400> 162
   tccttattcg ttgtcagtga ttg 23
<210> 163
   <211> 20
   <212> DNA
   <213> Homo sapiens
<400> 163
   catggtgaaa gacgatggac 20
<210> 164
   <211> 21
   <212> DNA
   <213> Homo sapiens
<400> 164
   tggggtaaag ggaatcaaaa g 21
<210> 165
   <211> 20
   <212> DNA
   <213> Homo sapiens
<400> 165
   ttgcatacat tcgaaagacc 20
<210> 166
   <211> 20
   <212> DNA
   <213> Homo sapiens
<400> 166
   cgtcagaaca agaccctgtg 20
<210> 167
   <211> 22
   <212> DNA
   <213> Homo sapiens
<400> 167
   cccggccact aagttatttt tc 22
<210> 168
   <211> 20
   <212> DNA
   <213> Homo sapiens
<400> 168
   ctgccattaa atgcgtcttg 20
<210> 169
   <211> 20
   <212> DNA
   <213> Homo sapiens
<400> 169
   ctttgggcct ttttcattcc 20
<210> 170
   <211> 20
   <212> DNA
   <213> Homo sapiens
<400> 170
   tgtctggctt atttcacacg 20
<210> 171
   <211> 24
   <212> DNA
   <213> Homo sapiens
<400> 171
   aacttttgac agcctactat gtgc 24
<210> 172
   <211> 23
   <212> DNA
   <213> Homo sapiens
<400> 172
   gaccattcat gaaagaaaca age 23
<210> 173
   <211> 23
   <212> DNA
   <213> Homo sapiens
<400> 173
   ccatgtaccg gtaacaaaag aag 23
<210> 174
   <211> 23
   <212> DNA
   <213> Homo sapiens
<400> 174
   tgagctttct aggategtae ctg 23
<210> 175
   <211> 20
   <212> DNA
   <213> Homo sapiens
<400> 175
   gcaggaaggt ccaacttgtc 20
<210> 176
   <211> 20
   <212> DNA
   <213> Homo sapiens
<400> 176
   atcttcaact gcgaacatgc 20
<210> 177
   <211> 26
   <212> DNA
   <213> Homo sapiens
<400> 177
   aagcatcaat gactacttta atcaac 26
<210> 178
   <211> 20
   <212> DNA
   <213> Homo sapiens
<400> 178
   tcccaaagtg ctgggattac 20
<210> 179
   <211> 20
   <212> DNA
   <213> Homo sapiens
<400> 179
   ttgttgcctt tgtcattttg 20
<210> 180
   <211> 20
   <212> DNA
   <213> Homo sapiens
<400> 180
   atgtgactgt gggcaggaac 20
<210> 181
   <211> 20
   <212> DNA
   <213> Homo sapiens
<400> 181
   gctggtgaga tgtcaaaacg 20
<210> 182
   <211> 26
   <212> DNA
   <213> Homo sapiens
<400> 182
   tcaacatatt acttcctcca gaactc 26
<210> 183
   <211> 20
   <212> DNA
   <213> Homo sapiens
<400> 183
   ttctcccatg tcagggaatc 20
<210> 184
   <211> 20
   <212> DNA
   <213> Homo sapiens
<400> 184
   gaccctcaaa ggctaacgtg 20
<210> 185
   <211> 21
   <212> DNA
   <213> Homo sapiens
<400> 185
   tccctggtca gcacagacta c 21
<210> 186
   <211> 21
   <212> DNA
   <213> Homo sapiens
<400> 186
   agctgtctca tttccaccat c 21
<210> 187
   <211> 20
   <212> DNA
   <213> Homo sapiens
<400> 187
   cgcgtcgttt atgtcaaatc 20
<210> 188
   <211> 20
   <212> DNA
   <213> Homo sapiens
<400> 188
   cgcgtcgttt atgtcaaatc 20
<210> 189
   <211> 20
   <212> DNA
   <213> Homo sapiens
<400> 189
   cataacacac aggggtgctg 20
<210> 190
   <211> 18
   <212> DNA
   <213> Homo sapiens
<400> 190
   gaactgggcg aggttgtg 18
<210> 191
   <211> 20
   <212> DNA
   <213> Homo sapiens
<400> 191
   tccctttctt acacgcaaac 20
<210> 192
   <211> 21
   <212> DNA
   <213> Homo sapiens
<400> 192
   cagttccgcc tgtacattca c 21
<210> 193
   <211> 19
   <212> DNA
   <213> Homo sapiens
<400> 193
   agcgctcctg ctttcagtc 19
<210> 194
   <211> 20
   <212> DNA
   <213> Homo sapiens
<400> 194
   gtcttggcgc agatcatcac 20
<210> 195
   <211> 21
   <212> DNA
   <213> Homo sapiens
<400> 195
   ttttgtcacc agttgaaatg c 21
<210> 196
   <211> 20
   <212> DNA
   <213> Homo sapiens
<400> 196
   gactgggaaa aagcatgagc 20
<210> 197
   <211> 25
   <212> DNA
   <213> Homo sapiens
<400> 197
   cggtgatcat aatattgtca ttgtg 25
<210> 198
   <211> 20
   <212> DNA
   <213> Homo sapiens
<400> 198
   ggaagtgtgg gcttgtcttc 20
<210> 199
   <211> 20
   <212> DNA
   <213> Homo sapiens
<400> 199
   tgcacagtcc atcctttgtc 20
<210> 200
   <211> 21
   <212> DNA
   <213> Homo sapiens
<400> 200
   aatgccagct ttcacaatgt c 21
<210> 201
   <211> 20
   <212> DNA
   <213> Homo sapiens
<400> 201
   ggccaagacc acatggtaag 20
<210> 202
   <211> 24
   <212> DNA
   <213> Homo sapiens
<400> 202
   tcctacatta agacagcatg gaac 24
<210> 203
   <211> 21
   <212> DNA
   <213> Homo sapiens
<400> 203
   tgcctccctt ttaaggctat c 21
<210> 204
   <211> 20
   <212> DNA
   <213> Homo sapiens
<400> 204
   aggtccttct gccaacaaag 20
<210> 205
   <211> 20
   <212> DNA
   <213> Homo sapiens
<400> 205
   cgtcttctct cctccaatgc 20
<210> 206
   <211> 20
   <212> DNA
   <213> Homo sapiens
<400> 206
   ggtattcagt tggggctcag 20
<210> 207
   <211> 20
   <212> DNA
   <213> Homo sapiens
<400> 207
   tgtatccacg tggtcagctc 20
<210> 208
   <211> 18
   <212> DNA
   <213> Homo sapiens
<400> 208
   acaggacgct cggtcaac 18
<210> 209
   <211> 25
   <212> DNA
   <213> Homo sapiens
<400> 209
   ttgccatcag tacaaatgag tttag 25
<210> 210
   <211> 24
   <212> DNA
   <213> Homo sapiens
<400> 210
   ttcctgcttt ttaagagtga tctg 24
<210> 211
   <211> 20
   <212> DNA
   <213> Homo sapiens
<400> 211
   aggaaggaag ggatggaaac 20
<210> 212
   <211> 18
   <212> DNA
   <213> Homo sapiens
<400> 212
   agaaaccact catgaaaa 18
<210> 213
   <211> 22
   <212> DNA
   <213> Homo sapiens
<400> 213
   cgcattacta catgatccac tg 22
<210> 214
   <211> 21
   <212> DNA
   <213> Homo sapiens
<400> 214
   tgtcacagaa agcatgagac c 21
<210> 215
   <211> 29
   <212> DNA
   <213> Homo sapiens
<400> 215
   agaaataact gtcaatatcc cagtatcac 29
<210> 216
   <211> 27
   <212> DNA
   <213> Homo sapiens
<400> 216
   tcattaaaca tttagtaatg tgtgctc 27
<210> 217
   <211> 19
   <212> DNA
   <213> Homo sapiens
<400> 217
   aggcaacagg gcaagactc 19
<210> 218
   <211> 21
   <212> DNA
   <213> Homo sapiens
<400> 218
   cctgaaaggg agaataaaag g 21
<210> 219
   <211> 21
   <212> DNA
   <213> Homo sapiens
<400> 219
   cctgaaaggg agaataaaag g 21
<210> 220
   <211> 20
   <212> DNA
   <213> Homo sapiens
<400> 220
   tattgaccca gccagcagac 20
<210> 221
   <211> 22
   <212> DNA
   <213> Homo sapiens
<400> 221
   tatattgaga ctcaaatatc ga 22
<210> 222
   <211> 21
   <212> DNA
   <213> Homo sapiens
<400> 222
   tgatgcatat cagagcgtga g 21
<210> 223
   <211> 21
   <212> DNA
   <213> Homo sapiens
<400> 223
   ttcaatgacc atgacaaaac g 21
<210> 224
   <211> 21
   <212> DNA
   <213> Homo sapiens
<400> 224
   ttcaatgacc atgacaaaac g 21
<210> 225
   <211> 21
   <212> DNA
   <213> Homo sapiens
<400> 225
   tggttttcaa gcagacaatc c 21
<210> 226
   <211> 21
   <212> DNA
   <213> Homo sapiens
<400> 226
   tcctcctcaa tgaaagcaga g 21
<210> 227
   <211> 20
   <212> DNA
   <213> Homo sapiens
<400> 227
   caatgtgatc ccaactggtc 20
<210> 228
   <211> 21
   <212> DNA
   <213> Homo sapiens
<400> 228
   ttattgccaa ttggagtttg g 21
<210> 229
   <211> 22
   <212> DNA
   <213> Homo sapiens
<400> 229
   ttctgttggc ttatcatttt tg 22
<210> 230
   <211> 23
   <212> DNA
   <213> Homo sapiens
<400> 230
   cccagaaact aaataaaatg cag 23
<210> 231
   <211> 25
   <212> DNA
   <213> Homo sapiens
<400> 231
   aatcaaattt gttgcattaa aaatc 25
<210> 232
   <211> 23
   <212> DNA
   <213> Homo sapiens
<400> 232
   gttttctcat tcctttctct tcc 23
<210> 233
   <211> 20
   <212> DNA
   <213> Homo sapiens
<400> 233
   tttgggaaag ggaacacaag 20
<210> 234
   <211> 22
   <212> DNA
   <213> Homo sapiens
<400> 234
   gatttttcct tggaacatcc tc 22
<210> 235
   <211> 20
   <212> DNA
   <213> Homo sapiens
<400> 235
   cggggatcag atttgctatg 20
<210> 236
   <211> 20
   <212> DNA
   <213> Homo sapiens
<400> 236
   tagggggtca tcctcaggtc 20
<210> 237
   <211> 20
   <212> DNA
   <213> Homo sapiens
<400> 237
   gtcttcccct gctcaatcac 20
<210> 238
   <211> 22
   <212> DNA
   <213> Homo sapiens
<400> 238
   gacacgttgt gggccagcca gt 22
<210> 239
   <211> 24
   <212> DNA
   <213> Homo sapiens
<400> 239
   ctgccggtta tcttcggaca cgtt 24
<210> 240
   <211> 20
   <212> DNA
   <213> Homo sapiens
<400> 240
   tgagtgaggg cagacagatg 20
<210> 241
   <211> 20
   <212> DNA
   <213> Homo sapiens
<400> 241
   tggcacctga accatgtaag 20
<210> 242
   <211> 20
   <212> DNA
   <213> Homo sapiens
<400> 242
   cgtacatgcc gaagtctgtc 20
<210> 243
   <211> 21
   <212> DNA
   <213> Homo sapiens
<400> 243
   gccctggttt taacccttaa c 21
<210> 244
   <211> 20
   <212> DNA
   <213> Homo sapiens
<400> 244
   ccagctccag cttctgactc 20
<210> 245
   <211> 20
   <212> DNA
   <213> Homo sapiens
<400> 245
   ttgtgttttc ttggagacag 20
<210> 246
   <211> 20
   <212> DNA
   <213> Homo sapiens
<400> 246
   caatgagcat gggagagatg 20
<210> 247
   <211> 21
   <212> DNA
   <213> Homo sapiens
<400> 247
   tggagtttct gggactacag g 21
<210> 248
   <211> 23
   <212> DNA
   <213> Homo sapiens
<400> 248
   ccttcttcaa agctgattct ctc 23
<210> 249
   <211> 20
   <212> DNA
   <213> Homo sapiens
<400> 249
   cgctctacag ccaatcacag 20
<210> 250
   <211> 20
   <212> DNA
   <213> Homo sapiens
<400> 250
   tggcatcaca atcaataggg 20
<210> 251
   <211> 20
   <212> DNA
   <213> Homo sapiens
<400> 251
   ctccaagggg gttagagtcc 20
<210> 252
   <211> 21
   <212> DNA
   <213> Homo sapiens
<400> 252
   caggaaacca ggtcagaagt g 21
<210> 253
   <211> 22
   <212> DNA
   <213> Homo sapiens
<400> 253
   tttttgcaga aaggggtctt ac 22
<210> 254
   <211> 20
   <212> DNA
   <213> Homo sapiens
<400> 254
   gcccacccca ctctagaaac 20
<210> 255
   <211> 21
   <212> DNA
   <213> Homo sapiens
<400> 255
   tggaaccttt tctgctcaaa g 21
<210> 256
   <211> 18
   <212> DNA
   <213> Homo sapiens
<400> 256
   agctgcatgg tgccaaag 18
<210> 257
   <211> 20
   <212> DNA
   <213> Homo sapiens
<400> 257
   ataacaatgg gcacatgcag 20
<210> 258
   <211> 21
   <212> DNA
   <213> Homo sapiens
<400> 258
   ggtcattctt ccatcagcaa g 21
<210> 259
   <211> 21
   <212> DNA
   <213> Homo sapiens
<400> 259
   cacacccaca ctcacacaaa g 21
<210> 260
   <211> 20
   <212> DNA
   <213> Homo sapiens
<400> 260
   ggcactgcag gctaataatg 20
<210> 261
   <211> 22
   <212> DNA
   <213> Homo sapiens
<400> 261
   gggacctcaa gtcttttcct tc 22
<210> 262
   <211> 22
   <212> DNA
   <213> Homo sapiens
<400> 262
   gggacctcaa gtcttttcct tc 22
<210> 263
   <211> 20
   <212> DNA
   <213> Homo sapiens
<400> 263
   ggaagggaag gaggacaaac 20
<210> 264
   <211> 22
   <212> DNA
   <213> Homo sapiens
<400> 264
   cgtctcaaac taccaagtct gg 22
<210> 265
   <211> 20
   <212> DNA
   <213> Homo sapiens
<400> 265
   cacccagtgc tgtttcaatg 20
<210> 266
   <211> 20
   <212> DNA
   <213> Homo sapiens
<400> 266
   cgccgcataa tgtgtaaaac 20
<210> 267
   <211> 22
   <212> DNA
   <213> Homo sapiens
<400> 267
   tgccatattt aactgccatt tc 22
<210> 268
   <211> 22
   <212> DNA
   <213> Homo sapiens
<400> 268
   tgccatattt aactgccatt tc 22
<210> 269
   <211> 24
   <212> DNA
   <213> Homo sapiens
<400> 269
   gcagtcactg agacagcttt tatc 24
<210> 270
   <211> 20
   <212> DNA
   <213> Homo sapiens
<400> 270
   taagcatagc ctcggagaac 20
<210> 271
   <211> 24
   <212> DNA
   <213> Homo sapiens
<400> 271
   ggaccattaa tagctacctt cctg 24
<210> 272
   <211> 23
   <212> DNA
   <213> Homo sapiens
<400> 272
   aggcaagaca acatatttga aag 23
<210> 273
   <211> 23
   <212> DNA
   <213> Homo sapiens
<400> 273
   aagggctatg tgtcattttg ttc 23
<210> 274
   <211> 21
   <212> DNA
   <213> Homo sapiens
<400> 274
   catcaagcaa gcaaacaaat g 21
<210> 275
   <211> 19
   <212> DNA
   <213> Homo sapiens
<400> 275
   aattccccca aaagcttcc 19
<210> 276
   <211> 20
   <212> DNA
   <213> Homo sapiens
<400> 276
   ttccctcctg gctaagaacc 20
<210> 277
   <211> 20
   <212> DNA
   <213> Homo sapiens
<400> 277
   aaaagcagag ggaatcatcg 20
<210> 278
   <211> 20
   <212> DNA
   <213> Homo sapiens
<400> 278
   tcccattcat gacctggaag 20
<210> 279
   <211> 20
   <212> DNA
   <213> Homo sapiens
<400> 279
   ggcccgcttt aagagatcag 20
<210> 280
   <211> 18
   <212> DNA
   <213> Homo sapiens
<400> 280
   catgcccaaa gtcgatcc 18
<210> 281
   <211> 20
   <212> DNA
   <213> Homo sapiens
<400> 281
   acacatccat ggtgttggtg 20
<210> 282
   <211> 20
   <212> DNA
   <213> Homo sapiens
<400> 282
   tgccacagcc acatagtctc 20
<210> 283
   <211> 22
   <212> DNA
   <213> Homo sapiens
<400> 283
   ttctatctgc agactcccac ag 22
<210> 284
   <211> 21
   <212> DNA
   <213> Homo sapiens
<400> 284
   ggaaaagaaa gcaggagaag c 21
<210> 285
   <211> 21
   <212> DNA
   <213> Homo sapiens
<400> 285
   aaatggagaa aagcctggtt c 21
<210> 286
   <211> 19
   <212> DNA
   <213> Homo sapiens
<400> 286
   aagcaatcct cccaccttg 19
<210> 287
   <211> 22
   <212> DNA
   <213> Homo sapiens
<400> 287
   ccttcctttt tcactcacac ac 22
<210> 288
   <211> 25
   <212> DNA
   <213> Homo sapiens
<400> 288
   tgatttaata atgaagatgg gttgg 25
<210> 289
   <211> 20
   <212> DNA
   <213> Homo sapiens
<400> 289
   actcagtacc ccaggcagag 20
<210> 290
   <211> 20
   <212> DNA
   <213> Homo sapiens
<400> 290
   tcaaactcct gggctcaaac 20
<210> 291
   <211> 18
   <212> DNA
   <213> Homo sapiens
<400> 291
   cagccacatc cccctatg 18
<210> 292
   <211> 20
   <212> DNA
   <213> Homo sapiens
<400> 292
   tgccttcttc cactcctttc 20
<210> 293
   <211> 23
   <212> DNA
   <213> Homo sapiens
<400> 293
   aagagtgaaa gcagagatgt tcc 23
<210> 294
   <211> 21
   <212> DNA
   <213> Homo sapiens
<400> 294
   actaagcctc aggagcagcc t 21
<210> 295
   <211> 25
   <212> DNA
   <213> Homo sapiens
<400> 295
   gatacttggg gaagagagac ctacc 25
<210> 296
   <211> 19
   <212> DNA
   <213> Homo sapiens
<400> 296
   gaggggagag gagggggag 19
<210> 297
   <211> 20
   <212> DNA
   <213> Homo sapiens
<400> 297
   cacaaacctg cccacattgc 20
<210> 298
   <211> 18
   <212> DNA
   <213> Homo sapiens
<400> 298
   cctgggcggc tcaactct 18
<210> 299
   <211> 19
   <212> DNA
   <213> Homo sapiens
<400> 299
   aggcgtttcc gtttatggc 19
<210> 300
   <211> 23
   <212> DNA
   <213> Homo sapiens
<400> 300
   ctgcttcttg agtaacactt acg 23
<210> 301
   <211> 26
   <212> DNA
   <213> Homo sapiens
<400> 301
   gattacgaag gtattggttt agacag 26
<210> 302
   <211> 26
   <212> DNA
   <213> Homo sapiens
<400> 302
   ggtgttaaaa atagttccat agttcg 26
<210> 303
   <211> 20
   <212> DNA
   <213> Homo sapiens
<400> 303
   tataagcagt ccctgccttc 20
<210> 304
   <211> 20
   <212> DNA
   <213> Homo sapiens
<400> 304
   tataagcagt ccctgccttc 20
<210> 305
   <211> 20
   <212> DNA
   <213> Homo sapiens
<400> 305
   ctgggcgaga gtgagattcc 20
<210> 306
   <211> 19
   <212> DNA
   <213> Homo sapiens
<400> 306
   atgaacccag gaggcagag 19
<210> 307
   <211> 20
   <212> DNA
   <213> Homo sapiens
<400> 307
   cggagatttg gatgttctcc 20
<210> 308
   <211> 20
   <212> DNA
   <213> Homo sapiens
<400> 308
   cggagatttg gatgttctcc 20
<210> 309
   <211> 21
   <212> DNA
   <213> Homo sapiens
<400> 309
   tttgtagaaa tggggtcttg c 21
<210> 310
   <211> 21
   <212> DNA
   <213> Homo sapiens
<400> 310
   aattcctgaa gctctcccaa g 21
<210> 311
   <211> 20
   <212> DNA
   <213> Homo sapiens
<400> 311
   tgctgaacca gtcaaactcc 20
<210> 312
   <211> 23
   <212> DNA
   <213> Homo sapiens
<400> 312
   ttgcaatatt ggtcctagag ttc 23
<210> 313
   <211> 26
   <212> DNA
   <213> Homo sapiens
<400> 313
   ccacaaatat caatttacaa ccattg 26
<210> 314
   <211> 25
   <212> DNA
   <213> Homo sapiens
<400> 314
   tggaaataat gttaagggtg ttttt 25
<210> 315
   <211> 20
   <212> DNA
   <213> Homo sapiens
<400> 315
   tctgcatggc cgatctaaag 20
<210> 316
   <211> 26
   <212> DNA
   <213> Homo sapiens
<400> 316
   aaagttgaga agctcatcac tggtac 26
<210> 317
   <211> 21
   <212> DNA
   <213> Homo sapiens
<400> 317
   tggttccaaa tcctaatctg c 21
<210> 318
   <211> 23
   <212> DNA
   <213> Homo sapiens
<400> 318
   ttgagggtag gagaatgaga gag 23
<210> 319
   <211> 22
   <212> DNA
   <213> Homo sapiens
<400> 319
   catgcatatt tcaaaggtca ag 22
<210> 320
   <211> 26
   <212> DNA
   <213> Homo sapiens
<400> 320
   tcaagtaaga ggaggatatg tcaaag 26
<210> 321
   <211> 24
   <212> DNA
   <213> Homo sapiens
<400> 321
   catcaaatat ttcaaaggtt gage 24
<210> 322
   <211> 20
   <212> DNA
   <213> Homo sapiens
<400> 322
   gtcaaaacaa atggcacacg 20
<210> 323
   <211> 20
   <212> DNA
   <213> Homo sapiens
<400> 323
   ttacaggcat gaaccaccac 20
<210> 324
   <211> 20
   <212> DNA
   <213> Homo sapiens
<400> 324
   cctatgcaat cggtctttgc 20
<210> 325
   <211> 21
   <212> DNA
   <213> Homo sapiens
<400> 325
   ggggattttt gttttgtttt g 21
<210> 326
   <211> 20
   <212> DNA
   <213> Homo sapiens
<400> 326
   aaaggggaaa tgegtaggae 20
<210> 327
   <211> 20
   <212> DNA
   <213> Homo sapiens
<400> 327
   tcccaaagtg ctgggattac 20
<210> 328
   <211> 27
   <212> DNA
   <213> Homo sapiens
<400> 328
   ccagaactta aagtgaaatt taaaaag 27
<210> 329
   <211> 19
   <212> DNA
   <213> Homo sapiens
<400> 329
   gcgaggcaaa acacaaagc 19
<210> 330
   <211> 21
   <212> DNA
   <213> Homo sapiens
<400> 330
   ttggaaatgg ctgtacctca g 21
<210> 331
   <211> 19
   <212> DNA
   <213> Homo sapiens
<400> 331
   tacttgagca gcccacagg 19
<210> 332
   <211> 24
   <212> DNA
   <213> Homo sapiens
<400> 332
   aaaggaatga aagtggtttt tgtc 24
<210> 333
   <211> 23
   <212> DNA
   <213> Homo sapiens
<400> 333
   tgcaatgtaa tagttttcca agg 23
<210> 334
   <211> 22
   <212> DNA
   <213> Homo sapiens
<400> 334
   cagcaaatga actaagccac ag 22
<210> 335
   <211> 24
   <212> DNA
   <213> Homo sapiens
<400> 335
   tgctatacta tttgcccaca aaac 24
<210> 336
   <211> 24
   <212> DNA
   <213> Homo sapiens
<400> 336
   gaatgcattt attcagagat gagg 24
<210> 337
   <211> 21
   <212> DNA
   <213> Homo sapiens
<400> 337
   tgctagacac ttgctggtca c 21
<210> 338
   <211> 25
   <212> DNA
   <213> Homo sapiens
<400> 338
   ttgatattaa agttgcacaa actgc 25
<210> 339
   <211> 25
   <212> DNA
   <213> Homo sapiens
<400> 339
   tcaattgtgt gacatatcac ctacc 25
<210> 340
   <211> 24
   <212> DNA
   <213> Homo sapiens
<400> 340
   tcactgtaga aatccaagta ccac 24
<210> 341
   <211> 21
   <212> DNA
   <213> Homo sapiens
<400> 341
   tctgcatcag tttgattctg c 21
<210> 342
   <211> 22
   <212> DNA
   <213> Homo sapiens
<400> 342
   aatgcacttt ttattttatt ag 22
<210> 343
   <211> 20
   <212> DNA
   <213> Homo sapiens
<400> 343
   gaaaagtgcc ggttcttgag 20
<210> 344
   <211> 20
   <212> DNA
   <213> Homo sapiens
<400> 344
   gcctacacag tccgttttcc 20
<210> 345
   <211> 19
   <212> DNA
   <213> Homo sapiens
<400> 345
   agaggagcgt gtgttgcag 19
<210> 346
   <211> 20
   <212> DNA
   <213> Homo sapiens
<400> 346
   actctgacgg tggagctgag 20
<210> 347
   <211> 24
   <212> DNA
   <213> Homo sapiens
<400> 347
   gctcttggtg ctaagttaaa gagg 24
<210> 348
   <211> 20
   <212> DNA
   <213> Homo sapiens
<400> 348
   atccagctgg ctctgatagg 20
<210> 349
   <211> 20
   <212> DNA
   <213> Homo sapiens
<400> 349
   tgaacagcca gatcctctcc 20
<210> 350
   <211> 20
   <212> DNA
   <213> Homo sapiens
<400> 350
   gtcccacctt gttaggaagc 20
<210> 351
   <211> 20
   <212> DNA
   <213> Homo sapiens
<400> 351
   tggcattctg aaaacggttc 20
<210> 352
   <211> 19
   <212> DNA
   <213> Homo sapiens
<400> 352
   gcaaacagcc tggacaatc 19
<210> 353
   <211> 22
   <212> DNA
   <213> Homo sapiens
<400> 353
   cacatatttc tgtcccctgt tg 22
<210> 354
   <211> 20
   <212> DNA
   <213> Homo sapiens
<400> 354
   tgtggttctt tggagcacag 20
<210> 355
   <211> 21
   <212> DNA
   <213> Homo sapiens
<400> 355
   ccaaggtaca tttcggaaaa c 21
<210> 356
   <211> 20
   <212> DNA
   <213> Homo sapiens
<400> 356
   accagccctt tcctcttgtc 20
<210> 357
   <211> 20
   <212> DNA
   <213> Homo sapiens
<400> 357
   ttcttcctca tgccattgtg 20
<210> 358
   <211> 19
   <212> DNA
   <213> Homo sapiens
<400> 358
   gtggcatctg gctgtcatc 19
<210> 359
   <211> 24
   <212> DNA
   <213> Homo sapiens
<400> 359
   caattagttt tccttgagca ctcc 24
<210> 360
   <211> 24
   <212> DNA
   <213> Homo sapiens
<400> 360
   tcttctttat ccaggacatc tgtg 24
<210> 361
   <211> 19
   <212> DNA
   <213> Homo sapiens
<400> 361
   cctgggagag gtctggttc 19
<210> 362
   <211> 20
   <212> DNA
   <213> Homo sapiens
<400> 362
   ggcagcatct tggtctgaag 20
<210> 363
   <211> 20
   <212> DNA
   <213> Homo sapiens
<400> 363
   gagcacttgg gagacctgag 20
<210> 364
   <211> 20
   <212> DNA
   <213> Homo sapiens
<400> 364
   agggaagcat gagcacagtc 20
<210> 365
   <211> 20
   <212> DNA
   <213> Homo sapiens
<400> 365
   tgagttctgt ctggctgtgg 20
<210> 366
   <211> 20
   <212> DNA
   <213> Homo sapiens
<400> 366
   tgatgaggga tgagggaaac 20
<210> 367
   <211> 20
   <212> DNA
   <213> Homo sapiens
<400> 367
   agggttaggg agcctagctg 20
<210> 368
   <211> 19
   <212> DNA
   <213> Homo sapiens
<400> 368
   tccttggaac acccctgtc 19
<210> 369
   <211> 25
   <212> DNA
   <213> Homo sapiens
<400> 369
   cagtcatgat acctacactt ccatc 25
<210> 370
   <211> 25
   <212> DNA
   <213> Homo sapiens
<400> 370
   caactctgaa ataaaagcaa tctgg 25
<210> 371
   <211> 25
   <212> DNA
   <213> Homo sapiens
<400> 371
   ttctttggtt atgaaatgaa caatc 25
<210> 372
   <211> 27
   <212> DNA
   <213> Homo sapiens
<400> 372
   ttgaataaaa gtagatgttt cttgtcc 27
<210> 373
   <211> 27
   <212> DNA
   <213> Homo sapiens
<400> 373
   taccaagaat ataatacgtt gttatgg 27
<210> 374
   <211> 20
   <212> DNA
   <213> Homo sapiens
<400> 374
   cggcttctgg cacataaaac 20
<210> 375
   <211> 23
   <212> DNA
   <213> Homo sapiens
<400> 375
   ccattgagca ctccattcat tac 23
<210> 376
   <211> 21
   <212> DNA
   <213> Homo sapiens
<400> 376
   ccctgggaat ctgaaagaat g 21
<210> 377
   <211> 20
   <212> DNA
   <213> Homo sapiens
<400> 377
   tgggccgttg tctcatatac 20
<210> 378
   <211> 20
   <212> DNA
   <213> Homo sapiens
<400> 378
   cactctggct tttccctctg 20
<210> 379
   <211> 20
   <212> DNA
   <213> Homo sapiens
<400> 379
   aggtcatgaa tgggatcctg 20
<210> 380
   <211> 19
   <212> DNA
   <213> Homo sapiens
<400> 380
   catattgctt ggcgtccac 19
<210> 381
   <211> 21
   <212> DNA
   <213> Homo sapiens
<400> 381
   tcttggtgat ctttgccttt g 21
<210> 382
   <211> 27
   <212> DNA
   <213> Homo sapiens
<400> 382
   tcatcaagat tattcgatat ttgagtc 27
<210> 383
   <211> 22
   <212> DNA
   <213> Homo sapiens
<400> 383
   cgagaaagta aagtgcctgc tg 22
<210> 384
   <211> 20
   <212> DNA
   <213> Homo sapiens
<400> 384
   cgggattgga gacagacatc 20
<210> 385
   <211> 19
   <212> DNA
   <213> Homo sapiens
<400> 385
   gaggatgctg ccatttgtg 19
<210> 386
   <211> 23
   <212> DNA
   <213> Homo sapiens
<400> 386
   catgctaaca gagtgtcaag agc 23
<210> 387
   <211> 20
   <212> DNA
   <213> Homo sapiens
<400> 387
   cgaattcttt ttgccatttc 20
<210> 388
   <211> 20
   <212> DNA
   <213> Homo sapiens
<400> 388
   aaagtctgca aggggctatg 20
<210> 389
   <211> 23
   <212> DNA
   <213> Homo sapiens
<400> 389
   tcaggctaga aatgtatcca agg 23
<210> 390
   <211> 20
   <212> DNA
   <213> Homo sapiens
<400> 390
   aaaggaaagg ggtaatccag 20
<210> 391
   <211> 27
   <212> DNA
   <213> Homo sapiens
<400> 391
   tttacttttt atgattacct ctgatgc 27
<210> 392
   <211> 27
   <212> DNA
   <213> Homo sapiens
<400> 392
   aaagaaaatt caaatgaaaa taagtcg 27
<210> 393
   <211> 22
   <212> DNA
   <213> Homo sapiens
<400> 393
   catgcaaact tgggtctaga tg 22
<210> 394
   <211> 20
   <212> DNA
   <213> Homo sapiens
<400> 394
   ttggcttttt cccctcatac 20
<210> 395
   <211> 20
   <212> DNA
   <213> Homo sapiens
<400> 395
   taaagccttt cccagctcag 20
<210> 396
   <211> 19
   <212> DNA
   <213> Homo sapiens
<400> 396
   cctgctgctt ccacaggac 19
<210> 397
   <211> 19
   <212> DNA
   <213> Homo sapiens
<400> 397
   catggacgtc ctgtggaag 19
<210> 398
   <211> 20
   <212> DNA
   <213> Homo sapiens
<400> 398
   gtgtcccatt catcctcacc 20
<210> 399
   <211> 19
   <212> DNA
   <213> Homo sapiens
<400> 399
   aacagaggag gcgctgaag 19
<210> 400
   <211> 18
   <212> DNA
   <213> Homo sapiens
<400> 400
   gcctcaccct acccatcc 18
<210> 401
   <211> 20
   <212> DNA
   <213> Homo sapiens
<400> 401
   agattgctgg ggttcctttc 20
<210> 402
   <211> 20
   <212> DNA
   <213> Homo sapiens
<400> 402
   ccacctcact ccatctctgg 20
<210> 403
   <211> 20
   <212> DNA
   <213> Homo sapiens
<400> 403
   tggggtaagt tccctgagtg 20
<210> 404
   <211> 20
   <212> DNA
   <213> Homo sapiens
<400> 404
   tacagagcca gggagagtgc 20
<210> 405
   <211> 20
   <212> DNA
   <213> Homo sapiens
<400> 405
   tatcatccac atcggtcagc 20
<210> 406
   <211> 25
   <212> DNA
   <213> Homo sapiens
<400> 406
   tttgggacaa gtaattgtta ttagc 25
<210> 407
   <211> 20
   <212> DNA
   <213> Homo sapiens
<400> 407
   ttgaatgcag tggtgctctc 20
<210> 408
   <211> 20
   <212> DNA
   <213> Homo sapiens
<400> 408
   tctgcctgtg ttctgagctg 20
<210> 409
   <211> 20
   <212> DNA
   <213> Homo sapiens
<400> 409
   gaactcagct ctgcctggac 20
<210> 410
   <211> 20
   <212> DNA
   <213> Homo sapiens
<400> 410
   gcgagactcg gtctcaaaag 20
<210> 411
   <211> 20
   <212> DNA
   <213> Homo sapiens
<400> 411
   atcgtttgcc aactcctagc 20
<210> 412
   <211> 20
   <212> DNA
   <213> Homo sapiens
<400> 412
   aatcagtgca ggtgatgcag 20
<210> 413
   <211> 20
   <212> DNA
   <213> Homo sapiens
<400> 413
   acatggcctg tgtctgcttc 20
<210> 414
   <211> 25
   <212> DNA
   <213> Homo sapiens
<400> 414
   gactggaaga aaataaccaa gtttc 25
<210> 415
   <211> 20
   <212> DNA
   <213> Homo sapiens
<400> 415
   ggcaggcgtt aaaggaatag 20
<210> 416
   <211> 19
   <212> DNA
   <213> Homo sapiens
<400> 416
   aaaaacaggg cacccattg 19
<210> 417
   <211> 20
   <212> DNA
   <213> Homo sapiens
<400> 417
   ttaagcccac agggaacaag 20
<210> 418
   <211> 20
   <212> DNA
   <213> Homo sapiens
<400> 418
   tgtcagacct tggccttttc 20
<210> 419
   <211> 23
   <212> DNA
   <213> Homo sapiens
<400> 419
   tcttctgaaa aatggaggaa gtc 23
<210> 420
   <211> 20
   <212> DNA
   <213> Homo sapiens
<400> 420
   gctcttcctg gggaagtctc 20
<210> 421
   <211> 20
   <212> DNA
   <213> Homo sapiens
<400> 421
   cagtttttga ctgccactgc 20
<210> 422
   <211> 21
   <212> DNA
   <213> Homo sapiens
<400> 422
   tccatgctcg acactattct g 21
<210> 423
   <211> 26
   <212> DNA
   <213> Homo sapiens
<400> 423
   ttctacttta catacaaaag gcactc 26
<210> 424
   <211> 20
   <212> DNA
   <213> Homo sapiens
<400> 424
   agttgggctt agcctggatg 20
<210> 425
   <211> 21
   <212> DNA
   <213> Homo sapiens
<400> 425
   agtatcacgt ccatgttgga g 21
<210> 426
   <211> 21
   <212> DNA
   <213> Homo sapiens
<400> 426
   caatgtttgc tttgaaaaag g 21
<210> 427
   <211> 20
   <212> DNA
   <213> Homo sapiens
<400> 427
   tgagcaaaac ctgtggaatg 20
<210> 428
   <211> 20
   <212> DNA
   <213> Homo sapiens
<400> 428
   tttgctggtg ctgtctatgg 20
<210> 429
   <211> 22
   <212> DNA
   <213> Homo sapiens
<400> 429
   ggatgtgcaa aatgttcttc tg 22
<210> 430
   <211> 21
   <212> DNA
   <213> Homo sapiens
<400> 430
   gggagcaggt gttattgatt g 21
<210> 431
   <211> 20
   <212> DNA
   <213> Homo sapiens
<400> 431
   ggtgaggagt tttcccaagc 20
<210> 432
   <211> 26
   <212> DNA
   <213> Homo sapiens
<400> 432
   agcacagagt ttgttaatgt ttttag 26
<210> 433
   <211> 23
   <212> DNA
   <213> Homo sapiens
<400> 433
   gctgacttct attgggagca tac 23
<210> 434
   <211> 21
   <212> DNA
   <213> Homo sapiens
<400> 434
   cagaggtatg gtttgggtct c 21
<210> 435
   <211> 21
   <212> DNA
   <213> Homo sapiens
<400> 435
   tgggggtcta ggactatgga g 21
<210> 436
   <211> 26
   <212> DNA
   <213> Homo sapiens
<400> 436
   gctgtgtttt cttaatttcc tgtatg 26
<210> 437
   <211> 19
   <212> DNA
   <213> Homo sapiens
<400> 437
   cagcctcctg cagactttg 19
<210> 438
   <211> 20
   <212> DNA
   <213> Homo sapiens
<400> 438
   cattttggga aaggaggttc 20
<210> 439
   <211> 20
   <212> DNA
   <213> Homo sapiens
<400> 439
   cggtcagtat gacggtaggg 20
<210> 440
   <211> 20
   <212> DNA
   <213> Homo sapiens
<400> 440
   aggtcatgaa tgggatcctg 20
<210> 441
   <211> 20
   <212> DNA
   <213> Homo sapiens
<400> 441
   ggcgctaatc gtactgaaac 20
<210> 442
   <211> 20
   <212> DNA
   <213> Homo sapiens
<400> 442
   tatggtggcc atggagactg 20
<210> 443
   <211> 19
   <212> DNA
   <213> Homo sapiens
<400> 443
   aggagccctc ctttgattg 19
<210> 444
   <211> 20
   <212> DNA
   <213> Homo sapiens
<400> 444
   ggccagtggt atctgctgac 20
<210> 445
   <211> 24
   <212> DNA
   <213> Homo sapiens
<400> 445
   aagacaaaat cccaaataaa gcag 24
<210> 446
   <211> 20
   <212> DNA
   <213> Homo sapiens
<400> 446
   attggtttga gtgccctttg 20
<210> 447
   <211> 22
   <212> DNA
   <213> Homo sapiens
<400> 447
   aaaatgcttt gcactgactc tg 22
<210> 448
   <211> 25
   <212> DNA
   <213> Homo sapiens
<400> 448
   ttcatcttta ttgcccctat atctg 25
<210> 449
   <211> 26
   <212> DNA
   <213> Homo sapiens
<400> 449
   ttaaagatta taccaagtca gtggtc 26
<210> 450
   <211> 20
   <212> DNA
   <213> Homo sapiens
<400> 450
   catgtggttt cttgcctttg 20
<210> 451
   <211> 24
   <212> DNA
   <213> Homo sapiens
<400> 451
   aagcataggc tcagcatact acac 24
<210> 452
   <211> 22
   <212> DNA
   <213> Homo sapiens
<400> 452
   cccatcaact accatgtgac tg 22
<210> 453
   <211> 22
   <212> DNA
   <213> Homo sapiens
<400> 453
   ggtcctgttg tcagtttttc ag 22
<210> 454
   <211> 20
   <212> DNA
   <213> Homo sapiens
<400> 454
   ggtcctgggg tgctcctaga 20
<210> 455
   <211> 22
   <212> DNA
   <213> Homo sapiens
<400> 455
   tcctcaactg agccaagtag cc 22
<210> 456
   <211> 22
   <212> DNA
   <213> Homo sapiens
<400> 456
   tgtgtcctcc atgttctgtt gg 22
<210> 457
   <211> 18
   <212> DNA
   <213> Homo sapiens
<400> 457
   tggcccctct gcctagca 18
<210> 458
   <211> 18
   <212> DNA
   <213> Homo sapiens
<400> 458
   ccactgctgg gtcctggg 18
<210> 459
   <211> 25
   <212> DNA
   <213> Homo sapiens
<400> 459
   gaatagagag cttttcctga gatgc 25
<210> 460
   <211> 24
   <212> DNA
   <213> Homo sapiens
<400> 460
   gattcatctt gaagaagttg atgg 24
<210> 461
   <211> 20
   <212> DNA
   <213> Homo sapiens
<400> 461
   acttgatgcc cccaagaatc 20
<210> 462
   <211> 21
   <212> DNA
   <213> Homo sapiens
<400> 462
   ctcaagaagc agaaagggaa g 21
<210> 463
   <211> 21
   <212> DNA
   <213> Homo sapiens
<400> 463
   tctacagagt tccctgtttg c 21
<210> 464
   <211> 21
   <212> DNA
   <213> Homo sapiens
<400> 464
   gctgtggatc ttagggacct c 21
<210> 465
   <211> 26
   <212> DNA
   <213> Homo sapiens
<400> 465
   aaaaagcatt tctgatatgg ataaag 26
<210> 466
   <211> 23
   <212> DNA
   <213> Homo sapiens
<400> 466
   tcgaagtatg ttgctatcct ctg 23
<210> 467
   <211> 25
   <212> DNA
   <213> Homo sapiens
<400> 467
   aaaataataa gcatcagcat ttgac 25
<210> 468
   <211> 21
   <212> DNA
   <213> Homo sapiens
<400> 468
   ttattccaga cgcatttcca c 21
<210> 469
   <211> 22
   <212> DNA
   <213> Homo sapiens
<400> 469
   tttgagtcta tcgagtgtgt gc 22
<210> 470
   <211> 21
   <212> DNA
   <213> Homo sapiens
<400> 470
   ttcctgtttt tcgtttggtt g 21
<210> 471
   <211> 21
   <212> DNA
   <213> Homo sapiens
<400> 471
   tgaattttcc ttttggggaa g 21
<210> 472
   <211> 25
   <212> DNA
   <213> Homo sapiens
<400> 472
   tggatcaaat ccaaataaag taagg 25
<210> 473
   <211> 25
   <212> DNA
   <213> Homo sapiens
<400> 473
   ttgctttttc tgtaaatcat ctgtg 25
<210> 474
   <211> 22
   <212> DNA
   <213> Homo sapiens
<400> 474
   tatttcattt atttatgtgg ac 22
<210> 475
   <211> 25
   <212> DNA
   <213> Homo sapiens
<400> 475
   gaagttaagg cagtgtttta gatgg 25
<210> 476
   <211> 25
   <212> DNA
   <213> Homo sapiens
<400> 476
   accagtaata tccactttct ttctg 25
<210> 477
   <211> 24
   <212> DNA
   <213> Homo sapiens
<400> 477
   tttattggat ttcaaaaatg agtg 24
<210> 478
   <211> 25
   <212> DNA
   <213> Homo sapiens
<400> 478
   tctcatgtga gaaagagatt agcag 25
<210> 479
   <211> 22
   <212> DNA
   <213> Homo sapiens
<400> 479
   tggctttcag tagttttcat gg 22
<210> 480
   <211> 18
   <212> DNA
   <213> Homo sapiens
<400> 480
   catgtgatgg cgtgatcc 18
<210> 481
   <211> 22
   <212> DNA
   <213> Homo sapiens
<400> 481
   aggaatacac aaacaccgac ag 22
<210> 482
   <211> 21
   <212> DNA
   <213> Homo sapiens
<400> 482
   tgcaccctgt tttcttttct c 21
<210> 483
   <211> 23
   <212> DNA
   <213> Homo sapiens
<400> 483
   tggacaagta atggttttct ctg 23
<210> 484
   <211> 21
   <212> DNA
   <213> Homo sapiens
<400> 484
   tgacatttga gcaaagacct g 21
<210> 485
   <211> 20
   <212> DNA
   <213> Homo sapiens
<400> 485
   tttgttttgt tttgtttttt 20
<210> 486
   <211> 27
   <212> DNA
   <213> Homo sapiens
<400> 486
   ttacttatag gtttcaggag atgtgtt 27
<210> 487
   <211> 23
   <212> DNA
   <213> Homo sapiens
<400> 487
   gggtctttcg aatgtatgca atg 23